# EUROPEAN PATENT SPECIFICATION

(11) **EP 1 382 678 B1**
(45) Date of publication and mention of the grant of the patent: **27.08.2008**
(21) Application number: 02720601.0
(22) Date of filing: 25.04.2002
(51) Int. Cl.: C12N 15/12, C12N 5/10, C07K 14/705, C12N 1/15, C12N 1/19, C12N 1/21, C07K 16/28, C12P 21/02, C12Q 1/02, C12Q 1/68, G01N 33/53

(54) **GUANOSINE TRIPHOSPHATE-BINDING PROTEIN-COUPLED RECEPOTR PLACE 6002312 AND ITS GENE AND PRODUCTION AND USE OF THE SAME**
GUANOSINTRIPHOSPHAT-BINDUNGSPROTEIN-GEKOPPELTER REZEPTOR PLACE 6002312 UND SEIN GEN SOWIE DESSEN PRODUKTION UND VERWENDUNG
RECEPTEUR COUPLE A LA PROTEINE FIXANT LA GUANOSINE TRIPHOSPHATE, PLACE 6002312, GENE CORRESPONDANT, PRODUCTION ET UTILISATION

(30) Priority: 25.04.2001 JP 2001127836
(43) Date of publication of application: 21.01.2004
(73) Proprietor: Astellas Pharma Inc., Tokyo 103-8411 (JP)
(72) Inventor: SUGIYAMA, Tomoyasu, Sumida-ku, Tokyo 130-0003 (JP); MORIKAWA, Noriyuki, Astellas Pharma Inc., Tokyo 103-8411 (JP); WAKAMATSU, Ai, Kisarazu-shi, Chiba 292-0014 (JP); ODA, Tamaki, Tsukuba-shi, Ibaraki 305-0051 (JP); IRIE, Ryotaro, Saitama-shi, Saitama 336-0936 (JP); ISOGAI, Takao, Inashiki-gun, Ibaraki 300-0303 (JP); MASUHO, Yasuhiko, Koganei-shi, Tokyo 184-0011 (JP)
(74) Representative: Gille Hrabal Struck Neidlein Prop Roos
(86) International application number: PCT/JP2002/004153
(87) International publication number: WO 2002/088355

(56) References cited:
- WO-A-02/10387
- WO-A1-01/77325
- WO-A1-99/66041
- WO-A2-00/22131
- WO-A2-00/31258
- WO-A2-01/98330
- KOBAYASHI TAKASHI ET AL: "Cloning, RNA expression, and chromosomal location of a mouse histamine H2 receptor gene" GENOMICS, vol. 37, no. 3, 1996, pages 390-394, XP002276303 ISSN: 0888-7543

### SEQUENCE LISTING

<110> Helix Research Institute
<120> Novel G protein-coupled receptors PLACE6002312 and genes encoding them, and their production and use.
<130> H1-A0101P
<140>
   <141>
<150> JP 2001-127836
   <151> 2001-04-25
<160> 20
<170> PatentIn Ver. 2.1
<210> 1
   <211> 1869
   <212> DNA
   <213> Homo sapiens
<220>
   <221> CDS
   <222> (735)..(1727)
<400> 1
   cccaaccaga tcccgtggtt ctgaggaccg gccctggaac cctgcgtcct tccgacccac 60
   cagctccctc gaggatggac ttgaggctgg atgctttcct cttcccactt tttgtcccaa 120
   cagaagtccc tgatctccag gatccctgac tggctgtgca gagctggcac agccacactc 180
   cacgggcttc cagcccttca ggcccagctc tcccggagag caccagagtc cctttgctcc 240
   atttacttgc gctcttcagc cagcaagtgt ttcctaatgt ctcaccccca gtcccagctt 300
   tcctgctgca atttccgcct gttccctccc atttcaggcc ttaagggagt gtggctgggc 360
   ttctctcctc cccctcactc caaagccgca cttgatacaa ggtggaaatc aaagcaccac 420
   ttctggcttc ccagccttga acttgccctt ccctctcaat gtatctggtt tcctcagctt 480
   ccctgtgcag caagagtgtc actcccctct tgcttttccc aagctcccca tgcgcccctc 540
   tcagcagatc tgtcttagag gggccaggtg cagagaaagg cagaaatcct aataatgttc 600
   ccagcgatcg ctgcctgtcg cggagtgctg aagggccaag tgttgatctg agtcctcatc 660
   acacgggact tcatttagtc tcacgatagg catgccggct gccgctccag gactcccctg 720
   tccccaggac caag atg acg ccc aac agc act ggc gag gtg ccc agc ccc   770
                   Met Thr Pro Asn Ser Thr Gly Glu Val Pro Ser Pro
                   1               5                   10
   att ccc aag ggg gct ttg ggg ctc tcc ctg gcc ctg gca agc ctc atc   818
   Ile Pro Lys Gly Ala Leu Gly Leu Ser Leu Ala Leu Ala Ser Leu Ile
           15                  20                  25
   atc acc gcg aac ctg ctc cta gcc ctg ggc atc gcc tgg gac cgc cgc   866
   Ile Thr Ala Asn Leu Leu Leu Ala Leu Gly Ile Ala Trp Asp Arg Arg
       30                  35                  40
   ctg cgc agc cca cct gct ggc tgc ttc ttc ctg agc cta ctg ctg gct   914
   Leu Arg Ser Pro Pro Ala Gly Cys Phe Phe Leu Ser Leu Leu Leu Ala
   45 50 55 60
   ggg ctg ctc acg ggt ctg gca ttg ccc aca ttg cca ggg ctg tgg aac 962
   Gly Leu Leu Thr Gly Leu Ala Leu Pro Thr Leu Pro Gly Leu Trp Asn
   65 70 75
   cag agt cgc cgg ggt tac tgg tcc tgc ctc ctc gtc tac ttg gct ccc 1010
   Gln Ser Arg Arg Gly Tyr Trp Ser Cys Leu Leu Val Tyr Leu Ala Pro
   80 85 90
   aac ttc tcc ttc ctc tcc ctg ctt gcc aac ctc ttg ctg gtg cac ggg 1058
   Asn Phe Ser Phe Leu Ser Leu Leu Ala Asn Leu Leu Leu Val His Gly
   95 100 105
   gag cgc tac atg gca gtc ctg agg cca ctc cag ccc cct ggg agc att 1106
   Glu Arg Tyr Met Ala Val Leu Arg Pro Leu Gln Pro Pro Gly Ser Ile
   110 115 120
   cgg ctg gcc ctg ctc ctc acc tgg gct ggt ccc ctg ctc ttt gcc agt 1154
   Arg Leu Ala Leu Leu Leu Thr Trp Ala Gly Pro Leu Leu Phe Ala Ser
   125 130 135 140
   ctg ccc gct ctg ggg tgg aac cac tgg acc cct ggc gcc aac tgc agc 1202
   Leu Pro Ala Leu Gly Trp Asn His Trp Thr Pro Gly Ala Asn Cys Ser
   145 150 155
   tcc cag gct atc ttc cca gcc ccc tac ctg tac ctc gaa gtc tat ggg 1250
   Ser Gln Ala Ile Phe Pro Ala Pro Tyr Leu Tyr Leu Glu Val Tyr Gly
   160 165 170
   ctc ctg ctg ccc gcc gtg ggt gct gct gcc ttc ctc tct gtc cgc gtg 1298
   Leu Leu Leu Pro Ala Val Gly Ala Ala Ala Phe Leu Ser Val Arg Val
   175 180 185
   ctg gcc act gcc cac cgc cag ctg cag gac atc tgt cgg ctg gag cgg 1346
   Leu Ala Thr Ala His Arg Gln Leu Gln Asp Ile Cys Arg Leu Glu Arg
   190 195 200
   gca gtg tgc cgc gat gag ccc tcc gcc ctg gcc cgg gcc ctt acc tgg 1394
   Ala Val Cys Arg Asp Glu Pro Ser Ala Leu Ala Arg Ala Leu Thr Trp
   205 210 215 220
   agg cag gca agg gca cag gct gga gcc atg ctg ctc ttc ggg ctg tgc 1442
   Arg Gln Ala Arg Ala Gln Ala Gly Ala Met Leu Leu Phe Gly Leu Cys
   225 230 235
   tgg ggg ccc tac gtg gcc aca ctg ctc ctc tca gtc ctg gcc tat gag 1490
   Trp Gly Pro Tyr Val Ala Thr Leu Leu Leu Ser Val Leu Ala Tyr Glu
   240 245 250
   cag cgc ccg cca ctg ggg cct ggg aca ctg ttg tcc ctc ctc tcc ctg 1538
   Gln Arg Pro Pro Leu Gly Pro Gly Thr Leu Leu Ser Leu Leu Ser Leu
   255 260 265
   gga agt gcc agt gca gcg gca gtg ccc gta gcc atg ggg ctg ggc gat 1586
   Gly Ser Ala Ser Ala Ala Ala Val Pro Val Ala Met Gly Leu Gly Asp
   270 275 280
   cag cgc tac aca gcc ccc tgg agg gca gcc gcc caa agg tgc ctg cag 1634
   Gln Arg Tyr Thr Ala Pro Trp Arg Ala Ala Ala Gln Arg Cys Leu Gln
   285 290 295 300
   ggg ctg tgg gga aga gcc tcc cgg gac agt ccc ggc ccc agc att gcc 1682
   Gly Leu Trp Gly Arg Ala Ser Arg Asp Ser Pro Gly Pro Ser Ile Ala
   305 310 315
   tac cac cca agc agc caa agc agt gtc gac ctg gac ttg aac taa 1727
   Tyr His Pro Ser Ser Gln Ser Ser Val Asp Leu Asp Leu Asn
   320 325 330
   aggaagggcc tctgctgact cctaccagag catccgtcca gctcagccat ccagcctgtc 1787
   tctactgggc cccacttctc tggatcagag accctgcctc tgtttgaccc cgcactgact 1847
   gaataaagct cctctggccg tt 1869
<210> 2
   <211> 330
   <212> PRT
   <213> Homo sapiens
<400> 2
   Met Thr Pro Asn Ser Thr Gly Glu Val Pro Ser Pro Ile Pro Lys Gly
   1 5 10 15
   Ala Leu Gly Leu Ser Leu Ala Leu Ala Ser Leu Ile Ile Thr Ala Asn
   20 25 30
   Leu Leu Leu Ala Leu Gly Ile Ala Trp Asp Arg Arg Leu Arg Ser Pro
   35 40 45
   Pro Ala Gly Cys Phe Phe Leu Ser Leu Leu Leu Ala Gly Leu Leu Thr
   50 55 60
   Gly Leu Ala Leu Pro Thr Leu Pro Gly Leu Trp Asn Gln Ser Arg Arg
   65 70 75 80
   Gly Tyr Trp Ser Cys Leu Leu Val Tyr Leu Ala Pro Asn Phe Ser Phe
   85 90 95
   Leu Ser Leu Leu Ala Asn Leu Leu Leu Val His Gly Glu Arg Tyr Met
   100 105 110
   Ala Val Leu Arg Pro Leu Gln Pro Pro Gly Ser Ile Arg Leu Ala Leu
   115 120 125
   Leu Leu Thr Trp Ala Gly Pro Leu Leu Phe Ala Ser Leu Pro Ala Leu
   130 135 140
   Gly Trp Asn His Trp Thr Pro Gly Ala Asn Cys Ser Ser Gln Ala Ile
   145 150 155 160
   Phe Pro Ala Pro Tyr Leu Tyr Leu Glu Val Tyr Gly Leu Leu Leu Pro
   165 170 175
   Ala Val Gly Ala Ala Ala Phe Leu Ser Val Arg Val Leu Ala Thr Ala
   180 185 190
   His Arg Gln Leu Gln Asp Ile Cys Arg Leu Glu Arg Ala Val Cys Arg
   195 200 205
   Asp Glu Pro Ser Ala Leu Ala Arg Ala Leu Thr Trp Arg Gln Ala Arg
   210 215 220
   Ala Gln Ala Gly Ala Met Leu Leu Phe Gly Leu Cys Trp Gly Pro Tyr
   225 230 235 240
   Val Ala Thr Leu Leu Leu Ser Val Leu Ala Tyr Glu Gln Arg Pro Pro
   245 250 255
   Leu Gly Pro Gly Thr Leu Leu Ser Leu Leu Ser Leu Gly Ser Ala Ser
   260 265 270
   Ala Ala Ala Val Pro Val Ala Met Gly Leu Gly Asp Gln Arg Tyr Thr
   275 280 285
   Ala Pro Trp Arg Ala Ala Ala Gln Arg Cys Leu Gln Gly Leu Trp Gly
   290 295 300
   Arg Ala Ser Arg Asp Ser Pro Gly Pro Ser Ile Ala Tyr His Pro Ser
   305 310 315 320
   Ser Gln Ser Ser Val Asp Leu Asp Leu Asn
   325 330
<210> 3
   <211> 30
   <212> RNA
   <213> Artificial Sequence
<220>
   <223> Description of Artificial Sequence:an artificially synthesized oligo linker sequence
<400> 3
   agcaucgagu cggccuuguu ggccuacugg 30
<210> 4
   <211> 42
   <212> DNA
   <213> Artificial Sequence
<220>
   <223> Description of Artificial Sequence:an artificially synthesized oligo dT adapter primer sequence
<400> 4
   gcggctgaag acggcctatg tggccttttt tttttttttt tt 42
<210> 5
   <211> 21
   <212> DNA
   <213> Artificial Sequence
<220>
   <223> Description of Artificial Sequence:an artificially synthesized primer sequence
<400> 5
   agcatcgagt cggccttgtt g 21
<210> 6
   <211> 42
   <212> DNA
   <213> Artificial Sequence
<220>
   <223> Description of Artificial Sequence:an artificially synthesized primer sequence
<400> 6
   gcggctgaag acggcctatg tggccttttt tttttttttt tt 42
<210> 7
   <211> 990
   <212> DNA
   <213> Homo sapiens
<400> 7
   atgacgccca acagcactgg cgaggtgccc agccccattc ccaagggggc tttggggctc 60
   tccctggccc tggcaagcct catcatcacc gcgaacctgc tcctagccct gggcatcgcc 120
   tgggaccgcc gcctgcgcag cccacctgct ggctgcttct tcctgagcct actgctggct 180
   gggctgctca cgggtctggc attgcccaca ttgccagggc tgtggaacca gagtcgccgg 240
   ggttactggt cctgcctcct cgtctacttg gctcccaact tctccttcct ctccctgctt 300
   gccaacctct tgctggtgca cggggagcgc tacatggcag tcctgaggcc actccagccc 360
   cctgggagca ttcggctggc cctgctcctc acctgggctg gtcccctgct ctttgccagt 420
   ctgcccgctc tggggtggaa ccactggacc cctggcgcca actgcagctc ccaggctatc 480
   ttcccagccc cctacctgta cctcgaagtc tatgggctcc tgctgcccgc cgtgggtgct 540
   gctgccttcc tctctgtccg cgtgctggcc actgcccacc gccagctgca ggacatctgt 600
   cggctggagc gggcagtgtg ccgcgatgag ccctccgccc tggcccgggc ccttacctgg 660
   aggcaggcaa gggcacaggc tggagccatg ctgctcttcg ggctgtgctg ggggccctac 720
   gtggccacac tgctcctctc agtcctggcc tatgagcagc gcccgccact ggggcctggg 780
   acactgttgt ccctcctctc cctgggaagt gccagtgcag cggcagtgcc cgtagccatg 840
   gggctgggcg atcagcgcta cacagccccc tggagggcag ccgcccaaag gtgcctgcag 900
   gggctgtggg gaagagcctc ccgggacagt cccggcccca gcattgccta ccacccaagc 960
   agccaaagca gtgtcgacct ggacttgaac 990
<210> 8
   <211> 26
   <212> DNA
   <213> Artificial Sequence
<220>
   <223> Description of Artificial Sequence:an artificially synthesized primer sequence
<400> 8
   catggcagtc ctgaggccac tccagc 26
<210> 9
   <211> 26
   <212> DNA
   <213> Artificial Sequence
<220>
   <223> Description of Artificial Sequence:an artificially synthesized primer sequence
<400> 9
   aggcaccttt gggcggctgc cctcca 26
<210> 10
   <211> 528
   <212> DNA
   <213> Homo sapiens
<400> 10
   gctacatggc agtcctgagg ccactccagc cccctgggag cattcggctg gccctgctcc 60
   tcacctgggc tggtcccctg ctctttgcca gtctgcccgc tctggggtgg aaccactgga 120
   cccctggcgc caactgcagc tcccaggcta tcttcccagc cccctacctg tacctcgaag 180
   tctatgggct cctgctgccc gccgtgggtg ctgctgcctt cctctctgtc cgcgtgctgg 240
   ccactgccca ccgccagctg caggacatct gtcggctgga gcgggcagtg tgccgcgatg 300
   agccctccgc cctggcccgg gcccttacct ggaggcaggc aagggcacag gctggagcca 360
   tgctgctctt cgggctgtgc tgggggccct acgtggccac actgctcctc tcagtcctgg 420
   cctatgagca gcgcccgcca ctggggcctg ggacactgtt gtccctcctc tccctgggaa 480
   gtgccagtgc agcggcagtg cccgtagcca tggggctggg cgatcagc 528
<210> 11
   <211> 29
   <212> DNA
   <213> Artificial Sequence
<220>
   <223> Description of Artificial Sequence:an artificially synthesized primer sequence
<400> 11
   ggaattcatt tagtctcacg ataggcatg 29
<210> 12
   <211> 32
   <212> DNA
   <213> Artificial Sequence
<220>
   <223> Description of Artificial Sequence:an artificially synthesized primer sequence
<400> 12
   cgggatcctc agtgcggggt caaacagagg ca 32
<210> 13
   <211> 24
   <212> DNA
   <213> Artificial Sequence
<220>
   <223> Description of Artificial Sequence:an artificially synthesized primer sequence
<400> 13
   cagctcccag gctatcttcc cagc 24
<210> 14
   <211> 23
   <212> DNA
   <213> Artificial Sequence
<220>
   <223> Description of Artificial Sequence:an artificially synthesized primer sequence
<400> 14
   actcactata gggctcgagc ggc 23
<210> 15
   <211> 25
   <212> DNA
   <213> Artificial Sequence
<220>
   <223> Description of Artificial Sequence:an artificially synthesized primer sequence
<400> 15
   gcactcgtag acacctttgg gcagc 25
<210> 16
   <211> 27
   <212> DNA
   <213> Artificial Sequence
<220>
   <223> Description of Artificial Sequence:an artificially synthesized primer sequence
<400> 16
   ccatcctaat acgactcact atagggc 27
<210> 17
   <211> 24
   <212> DNA
   <213> Artificial Sequence
<220>
   <223> Description of Artificial Sequence:an artificially synthesized primer sequence
<400> 17
   atggcccgaa gacctgcaag agtg 24
<210> 18
   <211> 30
   <212> DNA
   <213> Artificial Sequence
<220>
   <223> Description of Artificial Sequence:an artificially synthesized primer sequence
<400> 18
   tggccagaga tttatttggt ctgggtaggt 30
<210> 19
   <211> 1237
   <212> DNA
   <213> Mus musculus
<220>
   <221> CDS
   <222> (131)..(1120)
<400> 19
   atggcccgaa gacctgcaag agtggctgat gtgactcttt gatccgcaga ggaagagcta 60
   gcaagcacca cctgctccta acagccatgc atgctttctg cagctccgga ccctccatgt 120
   gccaagaccc atg atg aca ccc aac agc act gag ctg tcg gcc att ccc 169
   Met Met Thr Pro Asn Ser Thr Glu Leu Ser Ala Ile Pro
   1 5 10
   atg ggg gtt ctg ggg ctt tcc ttg gcc ctg gca agc ctc atc gtc atc 217
   Met Gly Val Leu Gly Leu Ser Leu Ala Leu Ala Ser Leu Ile Val Ile
   15 20 25
   gcc aac ctg ctc ctg gcc cta ggc atc gcc ctg gac cgc cac ttg cgc 265
   Ala Asn Leu Leu Leu Ala Leu Gly Ile Ala Leu Asp Arg His Leu Arg
   30 35 40 45
   agc cca cct gct ggc tgc ttc ttc cta agc cta cta cta gcc ggg ctg 313
   Ser Pro Pro Ala Gly Cys Phe Phe Leu Ser Leu Leu Leu Ala Gly Leu
   50 55 60
   ctc aca ggg ctg gca ctg ccc atg ctg cct ggg cta tgg agc cgg aac 361
   Leu Thr Gly Leu Ala Leu Pro Met Leu Pro Gly Leu Trp Ser Arg Asn
   65 70 75
   cat cag ggc tac tgg tcc tgc ctc ctt ctc cac ttg acc ccc aac ttt 409
   His Gln Gly Tyr Trp Ser Cys Leu Leu Leu His Leu Thr Pro Asn Phe
   80 85 90
   tgt ttc ctt tcc ctg ctt gcc aat ctg ctg ctg gtg cat ggg gaa cgc 457
   Cys Phe Leu Ser Leu Leu Ala Asn Leu Leu Leu Val His Gly Glu Arg
   95 100 105
   tac atg gca gtg ttg cag cca ctc cgg ccc cat gga agt gtg cgg cta 505
   Tyr Met Ala Val Leu Gln Pro Leu Arg Pro His Gly Ser Val Arg Leu
   110 115 120 125
   gcc ctg ttc ctc acc tgg gtc agc tcc ctg ttc ttt gcc agc ctg cct 553
   Ala Leu Phe Leu Thr Trp Val Ser Ser Leu Phe Phe Ala Ser Leu Pro
   130 135 140
   gct ctg ggc tgg aac cat tgg agc cct gat gcc aac tgc agc tcc caa 601
   Ala Leu Gly Trp Asn His Trp Ser Pro Asp Ala Asn Cys Ser Ser Gln
   145 150 155
   gct gtc ttc cca gcc ccc tac ctc tac ctg gaa gtt tat ggc ctc ctg 649
   Ala Val Phe Pro Ala Pro Tyr Leu Tyr Leu Glu Val Tyr Gly Leu Leu
   160 165 170
   ttg cct gcc gtg ggg gcc act gcc ctt ctc tct gtc cgc gtg ttg gcc 697
   Leu Pro Ala Val Gly Ala Thr Ala Leu Leu Ser Val Arg Val Leu Ala
   175 180 185
   act gcc cac cgc cag ctg tgt gag atc cgc cga ctg gag cgg gca gtg 745
   Thr Ala His Arg Gln Leu Cys Glu Ile Arg Arg Leu Glu Arg Ala Val
   190 195 200 205
   tgc cgc gat gta ccc tca acc ctg gct agg gct ctc acc tgg agg cag 793
   Cys Arg Asp Val Pro Ser Thr Leu Ala Arg Ala Leu Thr Trp Arg Gln
   210 215 220
   gct agg gca cag gca gga gcc aca ctg ctc ttc ttg ctg tgt tgg ggg 841
   Ala Arg Ala Gln Ala Gly Ala Thr Leu Leu Phe Leu Leu Cys Trp Gly
   225 230 235
   ccc tat gtg gcc aca ttg ctc ctg tca gtc ttg gcc tat gag cgt cgc 889
   Pro Tyr Val Ala Thr Leu Leu Leu Ser Val Leu Ala Tyr Glu Arg Arg
   240 245 250
   cca cca cta ggg cct gga act ctg tta tcg ctc atc tca ttg ggc agc 937
   Pro Pro Leu Gly Pro Gly Thr Leu Leu Ser Leu Ile Ser Leu Gly Ser
   255 260 265
   acc agt gct gcc gct gtg cct gtg gcc atg ggg ctg ggt gat cag cgc 985
   Thr Ser Ala Ala Ala Val Pro Val Ala Met Gly Leu Gly Asp Gln Arg
   270 275 280 285
   tac aca gcc ccc tgg agg aca gct gcc caa agg tgt cta cga gtg ctt 1033
   Tyr Thr Ala Pro Trp Arg Thr Ala Ala Gln Arg Cys Leu Arg Val Leu
   290 295 300
   cga gga aga gcc aag agg gac aat cca ggc ccc agc act gcc tac cac 1081
   Arg Gly Arg Ala Lys Arg Asp Asn Pro Gly Pro Ser Thr Ala Tyr His
   305 310 315
   acc agt agc caa tgc agc att gac ctg gac ttg aat tag ggaagcagca 1130
   Thr Ser Ser Gln Cys Ser Ile Asp Leu Asp Leu Asn
   320 325 330
   gctactgctg cctcccagaa cactcttcta tctcacagtg ccccacttct ttggattgga 1190
   accctcactc catgagacct cacccagacc aaataaatct ctggcca 1237
<210> 20
   <211> 329
   <212> PRT
   <213> Mus musculus
<400> 20
   Met Met Thr Pro Asn Ser Thr Glu Leu Ser Ala Ile Pro Met Gly Val
   1 5 10 15
   Leu Gly Leu Ser Leu Ala Leu Ala Ser Leu Ile Val Ile Ala Asn Leu
   20 25 30
   Leu Leu Ala Leu Gly Ile Ala Leu Asp Arg His Leu Arg Ser Pro Pro
   35 40 45
   Ala Gly Cys Phe Phe Leu Ser Leu Leu Leu Ala Gly Leu Leu Thr Gly
   50 55 60
   Leu Ala Leu Pro Met Leu Pro Gly Leu Trp Ser Arg Asn His Gln Gly
   65 70 75 80
   Tyr Trp Ser Cys Leu Leu Leu His Leu Thr Pro Asn Phe Cys Phe Leu
   85 90 95
   Ser Leu Leu Ala Asn Leu Leu Leu Val His Gly Glu Arg Tyr Met Ala
   100 105 110
   Val Leu Gln Pro Leu Arg Pro His Gly Ser Val Arg Leu Ala Leu Phe
   115 120 125
   Leu Thr Trp Val Ser Ser Leu Phe Phe Ala Ser Leu Pro Ala Leu Gly
   130 135 140
   Trp Asn His Trp Ser Pro Asp Ala Asn Cys Ser Ser Gln Ala Val Phe
   145 150 155 160
   Pro Ala Pro Tyr Leu Tyr Leu Glu Val Tyr Gly Leu Leu Leu Pro Ala
   165 170 175
   Val Gly Ala Thr Ala Leu Leu Ser Val Arg Val Leu Ala Thr Ala His
   180 185 190
   Arg Gln Leu Cys Glu Ile Arg Arg Leu Glu Arg Ala Val Cys Arg Asp
   195 200 205
   Val Pro Ser Thr Leu Ala Arg Ala Leu Thr Trp Arg Gln Ala Arg Ala
   210 215 220
   Gln Ala Gly Ala Thr Leu Leu Phe Leu Leu Cys Trp Gly Pro Tyr Val
   225 230 235 240
   Ala Thr Leu Leu Leu Ser Val Leu Ala Tyr Glu Arg Arg Pro Pro Leu
   245 250 255
   Gly Pro Gly Thr Leu Leu Ser Leu Ile Ser Leu Gly Ser Thr Ser Ala
   260 265 270
   Ala Ala Val Pro Val Ala Met Gly Leu Gly Asp Gln Arg Tyr Thr Ala
   275 280 285
   Pro Trp Arg Thr Ala Ala Gln Arg Cys Leu Arg Val Leu Arg Gly Arg
   290 295 300
   Ala Lys Arg Asp Asn Pro Gly Pro Ser Thr Ala Tyr His Thr Ser Ser
   305 310 315 320
   Gln Cys Ser Ile Asp Leu Asp Leu Asn
   325

## Description

### Technical Field

The present invention relates to a novel polypeptide belonging to the G protein-coupled receptor family, a polynucleotide encoding the polypeptide, and production and use of these molecules.

### Background Art

Many medically important biological processes are mediated by proteins and/or second messengers (for example, it is well known that such processes are mediated by CAMP; Lefkowitz, Nature, 1991, 351: 353-354), which are involved in.signaling pathways in which G proteins participate (hereinafter, these proteins are referred to as "proteins associated with G proteins or the pathways involving G proteins"). Such proteins include, for example, G protein-coupled receptors for adrenergic agents and dopamine (Kobilka, B. K. et al. , Proc. Natl. Acad. Sci. USA, 1987, 84: 46-50; Kobilka, B. K. et al. , Science, 1987, 238: 650-656; Bunzow, J. R. et al., Nature, 1988, 336: 783-787); G proteins themselves; effector proteins (e.g., phospholipase C, adenylate cyclase, and phosphodiesterase); and actuator proteins (e.g., protein kinase A and protein kinase C; Simon, M. I. et al . , Science, 1991, 252: 802-8).

For example, signaling can be initiated when hormone binds to adenylate cyclase in a cell to activate the enzyme. The hormone-mediated enzyme activation depends on nucleotide GTP. GTP also influences the hormone binding. G protein tethers a hormone receptor to adenylate cyclase. When activated by the hormone receptor, G protein releases the bound GDP and then binds to GTP. The GTP-bound form subsequently binds to activated adenylate cyclase. Hydrolysis from GTP to GDP is catalyzed by G protein itself, and thus G protein returns to its basal inactive state. Thus, G protein plays two roles; one role is an intermediate that relays a signal from a receptor to an effector and another is a clock to regulate signal duration.

The members of the membrane protein gene superfamily of G protein-coupled receptors are characterized to contain seven putative transmembrane domains. These domains are thought to correspond to transmembrane α-helix connected together via extracellular loops and cytoplasmic loops. G protein-coupled receptors include many biologically active receptors, such as hormone receptors, viral receptors, growth factor receptors, and neural receptors.

G protein-coupled receptors (also known as "seven-transmembrane receptor") are characterized to contain seven conserved hydrophobic regions consisting of about 20 to 30 amino acids, which tether at least eight different hydrophilic loops. The G protein-coupled receptor family includes dopamine receptors which bind to neuroleptics used for the treatment of mental diseases and neuropathy. Other exemplary members of this family include, but are not limited to, receptors of calcitonin, adrenalin, endothelin, cAMP, adenosine, muscarine, acetylcholine, serotonin, histamine, thrombin, kinin, follicle-stimulating hormone, opsin, endothelial cell differentiation gene-1, rhodopsin, odorant, and cytomegalovirus.

Most G protein-coupled receptors have a conserved cysteine residue in each of the first two extracellular loops. Such cysteine residues form disulfide bonds that are expected to stabilize functional protein structures. The seven transmembrane regions are designated as TM1, TM2, TM3, TM4, TM5, TM6, and TM7. TM3 participates in signal transduction.

Phosphorylation and lipidation (palmitoylation or farnesylation) of the cysteine residues can influence signaling events mediated by some G protein-coupled receptors. Most G protein-coupled receptors contain phosphorylation sites within the third cytoplasmic loop and/or the carboxyl terminus. Some G protein-coupled receptors, such as β-adrenalin receptor, mediate receptor desensitization through phosphorylation by protein kinase A and/or a specific receptor kinase.

Ligand-binding sites in some G protein-coupled receptors comprise a hydrophilic socket formed by several G protein-coupled receptor transmembrane domains. This socket has been conceived to be surrounded by hydrophobic residues of a G protein-coupled receptor. It has been assumed that the hydrophilic surfaces of each G protein-coupled receptor transmembrane helix face inside and thus form a polar ligand-binding site. TM3 has a ligand-binding site such as TM3 aspartate residue, and thus participates in the ligand binding of some G protein-coupled receptors. Serine of TM5, asparagine of TM6, and phenylalanine or tyrosine of TM6 or TM7 also participate in the ligand-binding.

G protein-coupled receptors can bind to various intracellular enzymes, ion channels, and transporters via a heterotrimeric G protein in cells (See Johnson et al. Endoc. Rev. , 1989, 10: 317-331). The α-subunit of each G protein preferentially stimulates particular effectors, and thus modifies a variety of intracellular biological functions. Phosphorylation of intracellular residues of G protein-coupled receptors was identified as an important mechanism for regulating the coupling of some G protein-coupled receptors with G proteins. G protein-coupled receptors have been found in a wide variety of tissues in mammal hosts.

90% or more of pharmaceuticals, previously created by pharmaceutical manufacturers in the world, target extracellular interactions. The majority of such pharmaceuticals target G protein-coupled receptors and have been marketed and succeeded commercially. This fact shows that these receptors have a history as established and proven therapeutic targets.

One of G protein-coupled receptors that have drawn attention is histamine receptors. Histamine, which is contained in mast cells of every peripheral tissue, is a major mediator associated with inflammation, immunity, and allergy. Histamine also plays an important role in gastric-acid secretion from the gastric mucus membrane. In the brain, histamine is distributed in neurons and non-neural cells. Histamine-positive neuroplasms are present limitedly in the posterior lobe of the hypothalamus, but project on almost entire regions of the brain including the spinal cord and cerebral cortex. Histamine has been thought to participate in central nervous functions, such as arousal reaction, sexual behavior, and analgesia. These physiological activities of histamine have been thought to be mediated by three types of specific receptors, which are called H1, H2, and H3, respectively (Pharmacol. Rev. (1997) 49: 253). In addition, the existence of H4 has been reported recently (J. Biol. Chem. (2000) 275: 36781).

H1 receptor (GenBank accession No. D14436) is expressed in the brain, muscles of respiratory tract, gastrointestine and digestive organs, urogenital apparatus, circulatory organs, adrenal medulla, endothelial cells, lymphocytes, etc. H1 receptor studies have focused on H1 receptor in blood vessels and smooth muscles. Histamine induces contraction of smooth muscle. This event has been thought to be accompanied by a histamine-induced increase in intracellular Ca concentration, which is mediated by inositol 1,4,5-triphosphate (Eur. J. Pharmacol. (1987) 135: 69). In vascular endothelial cells, histamine induces: (1) a change in vascular permeability as a result of contraction of endothelial cells; (2) biosynthesis of prostacyclin; (3) production of platelet activating factor (PAF) ; (4) release of von Willebrand factor (VWF); and (5) synthesis of nitric oxide (NO). H1 receptor has been proven to be present in T lymphocytes (Gen. Pharmacol. (1996) 27: 289). In addition, it has also been reported that the physiological function of H1 receptor includes catecholamine release in the adrenal medulla (Biochem. Pharmacol. (1988) 37: 221); suppression of heart beat by the cardiac muscle (J. Pharmacol. Exp. Ther. (1990) 1: 71) ; and functions in the central nervous system (Agents Actions (1990) 30: 13). H1 receptor functions to increase the concentrations of intracellular inositol phosphate and calcium ion, mediated by G proteins belonging to the pertussis toxin-insensitive Gq/11 family (Br. J. Pharmacol. (1994) 112: 847)

H2 receptor (GenBank accession No. AB023486) has been reported to be expressed at high levels in the basal nuclei, almond nucleus, and cerebral cortex in the brain and expressed at low levels in the cerebellum and hypothalamus (J. Neurochem. (1992) 59: 290). H2 receptor is also expressed in the stomach and heart. A study using an antagonist specific to H2 receptor revealed that, in the stomach, H2 receptor played an important role in gastric-acid secretion (Br. J. Pharmacol. (1985) 86: 571). It has been reported that, in the heart, the H2 receptor-mediated histamine activity results in chronotropic and inotropic actions on the atrium and ventricle (J. Pharmacol. Exp. Ther. (1988) 246: 377). Furthermore, H2 receptor is known to participate in smooth muscle relaxation in the respiratory tract, uterus, and vascular smooth muscle (Br. J. Clin. Pharmacol. (1989) 27: 139) and function in the immune system (Pharmacol. Rev. (1990) 42: 45). It has been revealed that such intracellular signaling mediated by H2 receptor involve cAMP accumulation and increase in adenylyl cyclase activity (Br. J. Clin. Pharmacol. (1987) 91: 213).

H3 receptor is originally localized in histamine-containing neurons, and has been suggested to exist as a presynaptic receptor that controls the production and release of histamine (Nature (1983) 302: 832). In the mammalian brain, the histamine-containing neurons project to the entire region of cerebral cortex, and thus H3 receptor has been presumed to play an important role in the brain function (Trends Pharmacol. Sci. (1998) 19: 177). Furthermore, H3 receptor not only participates in histamine release but also regulates, in the pre-synapse, the release of a variety of neurotransmitters, such as acetylcholine, dopamine, γ-aminobutyric acid, glutamic acid, noradrenaline, and serotonin. In addition, H3 receptor is involved in peripheral neurotransmission in the digestive system, cardiovascular system, and respiratory tract (Pharmacol. Rev. (1997) 49: 253). The H3 receptor gene (GenBank accession No.NM007232) was identified in 1999 (Mol. Pharmacol. (1999) 55: 1101). While the overall homologies of this receptor to H1 and H2 receptors, are only about 22% and 20%, the regional homologies in the transmembrane region are 27% and 33%, respectively (Trends Pharmacol. Sci. (2000) 21: 11). It has been suggested that there are two subtypes, H3a and H3b, of H3 receptor, from experimentally different properties (Mol. Pharmacol. (1990) 38: 610). Furthermore, a previous report suggests that there is another receptor present in eosinophils, which is different in properties from the three histamine receptors described above and interacts with H3 receptor-specific agonists (Am. J. Respir. Crit. Care. Med. (1994) 149: 1506).

H4 receptor has about 30% homology to H3 receptor at the amino acid level. However, the expression profile of H4 receptor differs from that of H3 receptor. H4 receptor is reportedly undetectable in the brain, but expressed in peripheral blood lymphocytes, spleen, thymus, and small intestine (J. Biol. Chem. (2000) 275: 36781).

### Disclosure of the Invention

There is an apparent need to identify and characterize a new receptor as a target for preventing, ameriolating, and treating functional disorders or diseases including infection, such as bacterial infection, fungal infection, protozoiasis, and viral infection including HIV-1 or HIV-2 infection in particular, pain, cancer, diabetes, obesity, loss of appetite, hyperphagia, asthma, Parkinson's disease, acute heart failure, hypotension, hypertension, retention of urine, osteoporosis, stenocardia, myocardial infarction, ulcer, allergy, benign prostatic hypertrophy, and mental and neurologic disorders, including anxiety, schizophrenia, manic-depressive psychosis, delirium, dementia, severe mental deficiencies, such as Huntington's disease or Gilles de la Tourette's syndrome, and dyskinesia. The present invention provides such a receptor. The present invention provides in particular a novel histamine-binding G protein-coupled receptor, its gene, a method for producing them, and their uses.

The present inventors prepared a full-length cDNA library from human placental total RNA using the oligo-capping method and analyzed the structures of clones contained in the cDNA library. As a result, the inventors found that a clone referred to as PLACE6002312 contained hydrophobic domains that were assumed to be seven transmembrane domains characteristic of G protein-coupled receptors. In addition, the structure of PLACE6002312 contained some amino acid residues characteristic of G protein-coupled receptor whose ligand is monoamine or histamine, including the aspartic acid located immediately after the first transmembrane domain, DRY (or ERY) sequence located immediately after the third transmembrane domain, tryptophane in the fourth transmembrane domain, cysteine between the fourth and fifth transmembrane domains, and WXP sequence in the sixth transmembrane domain. These facts reveal that PLACE6002312 encodes a G protein-coupled receptor, as previously disclosed in Word Patent Application WO 02/10387. According to the database search, PLACE6002312 has the highest homology to histamine H2 receptor. Analysis for the distribution of PLACE 6002312 gene expression in human tissues revealed that the gene was expressed in various tissues, such as heart, liver, and ovary. In addition, a ligand-binding assay revealed that histamine is one of ligands of PLACE6002312. Using the ligand-binding assay system, screening can be performed for antagonists and agonists of PLACE6002312 that can be used as pharmaceuticals. Furthermore, since PLACE6002312 has been found to bind to histamine, it can be used to purify histamine.

Furthermore, the present inventors isolated a full-length cDNA of the mouse homolog of PLACE6002312 and found that the amino acid sequence deduced from its open reading frame comprises hydrophobic domains that were assumed to be seven transmembrane domains characteristic of G protein-coupled receptors.

PLACE6002312 is predicted to play important roles *in vivo*, and thus mutations in this gene and abnormal expression of this gene may cause various diseases. Such diseases include, for example, infection, such as bacterial infection, fungal infection, protozoiasis, and viral infection including HIV-1 or HIV-2 infection in particular, pain, cancer, diabetes, obesity, loss of appetite, hyperphagia asthma, Parkinson's disease, acute heart failure, hypotension, hypertension, retention of urine, osteoporosis, stenocardia, myocardial infarction, ulcer, allergy, benign prostatic hypertrophy, and mental and neurologic disorders, including anxiety, schizophrenia, manic-depressive psychosis, delirium, dementia, severe mental deficiencies, such as Huntington's disease or Gilles de la Tourette's syndrome, and dyskinesia. Inappropriate activity or expression level of PLACE6002312 can be utilized as important indicators for diagnosis of such diseases.

The present invention relates to a novel G protein-coupled receptor that binds to histamine, its gene, a method for producing them, and their uses. More specifically, it is provided the following (1) to (22).
(1) A guanosine triphosphate-binding protein-coupled receptor-encoding polynucleotide selected from any one of (a) through (d):
   (a) a polynucleotide which encodes a polypeptide comprising the amino acid sequence of SEQ ID NO: 2 or 20;
   (b) a polynucleotide which comprises a coding region of the nucleotide sequence of SEQ ID NO: 1 or 19;
   (c) a polynucleotide which encodes a polypeptide comprising the amino acid sequence of SEQ ID NO: 2 or 20 wherein one or more amino acids are substituted, deleted, added, and/or inserted; and
   (d) a polynucleotide which hybridizes to a DNA comprising the nucleotide sequence of SEQ ID NO: 1 or 19 under a stringent condition.
(2) A polynucleotide which encodes a fragment of a polypeptide comprising the amino acid sequence of SEQ ID NO: 2 or 20.
(3) The polynucleotide according to (1) or (2), wherein said polynucleotide encodes a polypeptide having histamine-binding activity.
(4) A vector which contains the polynucleotide according to any one of (1) to (3).
(5) A host cell which carries the polynucleotide according to any one of (1) to (3) or the vector according to (4).
(6) A polypeptide encoded by the polynucleotide according to any one of (1) to (3).
(7) A method for producing the polypeptide according to (6), which comprises the steps of culturing the host cell according to (5) and recovering the produced polypeptide from the culture supernatant of the host cell.
(8) A method for preparing a cell which produces the polypeptide according to (6), which comprises the step of transforming a host cell with the vector according to (4) to allow the host cell to produce the polypeptide according to (6) under an appropriate culture condition.
(9) The host cell according to (5) which expresses the polypeptide according to (6) on the cell surface, or the cell membrane thereof.
(10) An antibody which binds to the polypeptide according to (6).
(11) A method for identifying a ligand to the polypeptide according to (6), which comprises the steps of:
   (a) contacting the polypeptide according to (6) with a candidate compound; and
   (b) determining whether or not the candidate compound binds to the polypeptide according to (6).
(12) A method for identifying an agonist of the polypeptide according to (6), which comprises the steps of:
   (a) contacting a cell expressing the polypeptide according to (6) with a candidate compound; and
   (b) determining whether or not the candidate compound induces the generation of a signal that can be used as an indicator for the activation of the polypeptide according to (6).
(13) A method for identifying an antagonist of the polypeptide according to (6), which comprises the steps of:
   (a) contacting an agonist of the polypeptide according to (6) with a cell expressing the polypeptide according (6) in the presence of a candidate compound; and
   (b) determining whether or not a signal as an indicator for the activation of the polypeptide according to (6) is attenuated as compared with the case in the absence of the candidate compound.
(14) A ligand identified by the method according to (11).
(15) An agonist identified by the method according to (12).
(16) An antagonist identified by the method according to (13).
(17) A kit to be used in the methods according to any one of (11) to (13), which comprises at least one molecule selected from (a) through (c):
   (a) the polypeptide according to (6);
   (b) the host cell according to (9) or the cell membrane of the cell; and
   (c) the antibody according to (10).
(18) A pharmaceutical composition to be used for treating a patient who needs increased activity or an increased level of expression of the polypeptide according to (6), wherein said composition comprises a therapeutically effective amount of a molecule selected from (a) and' (b) :
   (a) an agonist of the polypeptide according to (6); and
   (b) the polynucleotide according to any one of (1) to (3), which is in a form that ensures the *in vivo* expression.
(19) A pharmaceutical composition to be used for treating a patient who needs decreased activity or a decreased level of expression of the polypeptide according to (6), which comprises a therapeutically effective amount of a molecule selected from (a) and (b):
   (a) an antagonist of the polypeptide according to (6); and
   (b) a polynucleotide which suppresses in vivo endogenous expression of a gene encoding the polypeptide according to (6).
(20) A method for diagnosing a disease which is associated with the expression of a gene encoding the polypeptide according to (6) or the activity of said polypeptide, which comprises the step of:
   (a) detecting mutations in a gene encoding the polypeptide according to (6) in the genome of a subject using a sample obtained from the subject; or
   (b) detecting the expression of a gene encoding the polypeptide according to (6) using the sample obtained from the subject.
(21) A kit to be used in the method according to (20), which comprises at least one molecule selected from (a) through (c):
   (a) the polynucleotide according to (1) or (2);
   (b) the polypeptide according to (6); and
   (c) the antibody according to (10).
(22) A composition to be used for inducing an immune response to the polypeptide according to (6) in mammals, which comprises a molecule selected from (a) and (b):
   (a) the polynucleotide according to (1) or (2); and
   (b) the polypeptide according to (6).

Various terms are defined below to clearly illustrate the present invention. However, the definitions described below are provided to aid the understanding of terms frequently used herein, but are not used to limit the present invention.

As used herein, the term "PLACE6002312" typically refers to a polypeptide comprising the amino acid sequence of SEQ ID NO: 2 or 20 or an allelic mutant of the polypeptide.

The phrases "receptor activity" and "biological activity of a receptor" refer to a metabolic or physiological function of PLACE6002312, including similar activities, enhanced activity, and such activities that accompany decreased undesirable side effects. Furthermore, these phrases also refer to antigenic and immunogenic activities of PLACE6002312 described above.

The term "PLACE6002312 gene" refers to a polynucleotide having the nucleotide sequence of SEQ ID NO: 1 or 19, or an allelic mutant of the polynucleotide, and/or the complimentary strand thereof.

The term "antibody" refers to polyclonal and monoclonal antibodies, chimeric antibody, single-stranded antibody, humanized antibody, and Fab or Fab fragment containing another product from an immunoglobulin expression library.

The term "isolated" refers to "modified artificially from the natural state". If an "isolated" composition or substance exists naturally, it must be different and/or transferred from the environment where it originally exists. For example, a naturally-occurring polynucleotide or polypeptide in a living animal body is not "isolated". When a polynucleotide or polypeptide is separated from substances coexisting in the natural state, it can be said to be "isolated" as used herein.

The term "polynucleotide" typically refers to any polyribonucleotide or polydeoxyribonucleotide, which may be an unmodified RNA or DNA, or modified RNA or DNA. The term "polynucleotide" includes, but is not limited to, a single-stranded and double-stranded DNA; a DNA comprising both single-stranded and double-stranded regions; a single-stranded and double-stranded RNA; an RNA comprising both single-stranded and double-stranded regions; a hybrid molecule comprising DNA and RNA (which may be single-stranded, more typically double-stranded, or may comprise both single-stranded and double-stranded regions). In addition, the term "polynucleotide" also refers to a triple-stranded region that may comprise either RNA or DNA, or both. The term "polynucleotide" also includes DNA or RNA containing one or more modified nucleotides, and DNA or RNA containing a backbone modified for stabilization or other reason. Such a "modified" nucleotide includes, for example, tritylated nucleotide and special nucleotide such as inosine. There are various modification techniques previously established to modify DNA, and RNA. Thus, the "polynucleotide" includes various forms obtained through chemical, enzymatic, or metabolic modification of a naturally occurring polynucleotide, and chemically modified forms of DNA and RNA characteristic of viruses and cells. The term "polynucleotide" also includes relatively short polynucleotides, which are often called "oligonucleotides".

The term "polypeptide" refers to any peptide or protein that comprises two or more amino acids linked together via peptide bond or modified peptide bond (namely, peptide isostere). The term "polypeptide" refers to both short chain (typically referred to as peptide, oligopeptide, or oligomer) and long chain (generally referred to as protein). A polypeptide may contain amino acids other than the 20 kinds of amino acids encoding the receptors. The term "polypeptide" may include amino acid sequences modified through a natural process, such as post-translational processing, or a chemical modification method known in the art. Such modifications are described in detail in basic textbooks, more detailed scientific papers, and research papers. A polypeptide can be modified at any part, including the peptide backbone, amino acid side chains, and amino and carboxyl.ends. The same modification may be made at several sites to a comparable degree or various degrees. Alternatively, a polypeptide may undergo various modifications.

A polypeptide may be branched due to ubiquitination, or may be a branched or non-branched ring. A cyclic, branched, or branched cyclic polypeptide can be produced in a spontaneous post-translational process, or by a synthetic method. Examples of polypeptide modifications include acetylation; acylation; ADP-ribosylation; amidation; covalent binding with flavin; covalent binding with heme moieties; covalent binding with nucleotides or nucleotide derivatives; covalent binding with lipids or lipid derivatives; covalent binding with phosphatidylinositols; cross-linkage; cyclization; disulfide bond formation; demethylation; covalent cross linkage formation; cystine formation pyroglutamate formation; formylation; γ-carboxylation; glycosylation; GPI-anchor formation; hydroxylation; iodination; methylation; myristoylation; oxidation; proteolytic treatment; phosphorylation; prenylation; racemization; selenoylation; sulfation; and transfer RNA-mediated amino acid addition to a protein such as arginylation; ubiquitination. See, for example, PROTEINS - STRUCTURE AND MOLECULAR PROPERTIES, 2nd Ed., T. E. Creighton, W. H. Freeman and Company, New York, 1993; Wold, F., Posttranslational Protein Modifications: Perspectives and Prospects, pgs.1-12 in POSTTRANSLATIONAL COVALENT MODIFICATION OF PROTEINS, (ed. B. C. Johnson), Academic Press, New York, 1983; Seifter et al. , "Analysis for protein modifications and nonprotein cofactors", Meth. Enzymol. (1990) 182: 626-646; and Rattan et al., "Protein Synthesis: Posttranslational Modifications and Aging", Ann NY Acad Sci (1992) 663: 48-62.

The term "mutant" refers to a polynucleotide or polypeptide that differs from the standard polynucleotide or polypeptide but retains the essential characteristics. A typical mutant of a polynucleotide differs from the standard polynucleotide in the nucleotide sequence. Such variations in the mutant nucleotide sequence may or may not change the amino acid sequence of the polypeptide encoded by the standard polynucleotide. As described below, the nucleotide alterations can result in amino acid substitution, addition, deletion, fusion, and terminal cleavage (truncation) in the polypeptide encoded by the standard sequence. A typical mutant polypeptide differs from its standard polypeptide at amino acid sequence. In general, such differences are limited so that the mutant sequence is closely similar to the standard polypeptide sequence as a whole and many regions in the two sequences match up. A mutant may differ from the standard polypeptide in the amino acid sequence by amino acid substitution, addition, deletion, or combination of two or more thereof. An amino acid residue to be substituted or inserted may or may not be encoded by a genetic code. A polynucleotide or polypeptide mutant may be a spontaneous mutant, such as allelic mutants, or a mutant that has not been found in nature. A non-spontaneous mutant of a polynucleotide or polypeptide can be prepared by mutagenesis or can be synthesized directly.

The term "identity" refers to a measure of nucleotide sequence identity or amino acid sequence identity. Generally, multiple sequences are aligned together so as to be maximally matched with (identical to) one another. The term "identity" itself has the art-recognized meaning, and can be determined using an established technique. See, for example, COMPUTATIONAL MOLECULARBIOLOGY, ed. Lesk, A. M. , Oxford University Press, New York, 1988; BIOCOMPUTING: INFORMATICS AND GENOMEPROJECTS, ed. Smith, D. W. , Academic Press, New York, 1993; COMPUTER ANALYSIS OF SEQUENCE DATA, PART I, eds. Griffin, A. M. and Griffin, H. G. , Humana Press, New Jersey, 1994; SEQUENCE ANALYSIS IN MOLECULAR BIOLOGY, von Heinje, G., Academic Press, 1987; and SEQUENCE ANALYSIS PRIMER, eds. Gribskov, M. and Devereux, J., M Stockton Press, New York, 1991. There are many methods for determining the identity between two polynucleotides or polypeptides. The term "identity" is known to those skilled in the art (Carillo, H. and Lipton, D. , SIAM J Applied Math (1988) 48: 1073).

Methods widely used to determine the identity or similarity between two sequences include, but are not limited to, the methods according to Guide to Huge Computers, ed. Martin J. Bishop, Academic Press, San Diego, 1994; and Carillo, H. and Lipton, D. , SIAM J Applied Math (1988) 48: 1073. Such methods for determining identity and similarity are integrated in some computer programs. Computer programs preferably used to determine identity or similarity between two sequences include, but are not limited to, GCS program package (Devereux, J. et al., Nucleic Acids Research (1984) 12 (1) : 387), BLASTP, BLASTN, and FASTA (Altschul, S. F. et al., J. Mol. Biol. (1990) 215: 403).

As an example, the phrase "a polynucleotide comprising a nucleotide sequence having, for example, at least 95% "identity" to the standard nucleotide sequence of SEQ ID NO: 1 or 19" means that the nucleotide sequence of the polynucleotide is identical to the standard nucleotide sequence of SEQ ID NO: 1 or 19, except that the nucleotide sequence can contain a maximum of five point mutations in every 100 nucleotides of the standard sequence. In other words, a polynucleotide comprising the nucleotide sequence at least 95% identical to the standard nucleotide sequence can be prepared by deleting up to 5% of nucleotides from the standard sequence, substituting up to 5% of nucleotides with other nucleotides, or inserting, into the standard sequence, nucleotides corresponding to up to 5% of entire nucleotides of the standard sequence. Such mutations in the standard sequence can be positioned at the 5' or 3' end of the standard nucleotide sequence, or between these ends, and may be made for each nucleotide in different positions in the standard nucleotide sequence or for one or more groups of consecutive residues in the standard sequence.

Similarly, the phrase "a polypeptide that comprises the amino acid sequence having, for example, at least 95% "identity" to the standard amino acid sequence of SEQ ID NO: 2 or 20" means that the polypeptide sequence is identical to the standard nucleotide sequence of SEQ ID NO: 2 or 20, except that the polypeptide sequence can contain a maximum of five amino acid alternations in every 100 amino acids of the standard sequence. In other words, a polypeptide comprising the amino acid sequence at least 95% identical to the standard amino acid sequence can be prepared by deleting up to 5% of the amino acid residues from the standard sequence, substituting up to 5% of the amino acid residues with other amino acids, or inserting, into the standard sequence, amino acids corresponding to up to 5% of entire amino acid residues in the standard sequence. Such alternations in the standard sequence can be positioned at the amino or carboxyl terminus of the standard amino acid sequence, or between these termini, and may be made for each amino acid in different positions in the standard sequence or for one or more groups of consecutive residues in the standard sequence.

The term "ligand" refers to molecules that bind to a polypeptide herein described, including both natural and synthetic ligands. "Agonist" refers to molecules that bind to and activate a polypeptide herein described. On the other hand, "antagonist" refers to molecules that inhibit the activation of a polypeptide herein described.

### <Polypeptide>

It is provided a novel polypeptide belonging to the G protein-coupled receptor family. The polypeptide herein described includes a polypeptide consisting of the amino acid sequence of SEQ ID NO: 2 or 20 and a polypeptide comprising the amino acid sequence of SEQ ID NO: 2 or 20 . The human PLACE6002312 protein encoded by cDNA having the nucleotide sequence of SEQ ID NO: 1, is structurally related to other proteins belonging to the G protein-coupled receptor family. The cDNA of SEQ ID NO: 1 contains the open reading frame (position 735 to 1724 nucleotide) of SEQ ID NO: 2 that encodes the polypeptide consisting of 330 amino acids. The amino acid sequence of SEQ ID NO: 2, which consists of 330 amino acid residues, is about 27% identical (estimated by using BLAST) to that of human histamine H2 receptor (GenBank accession No. P97292). Furthermore, the mouse PLACE6002312 homolog protein encoded by cDNA having the nucleotide sequence of SEQ ID NO: 19, is structurally related to other proteins belonging to the G protein-coupled receptor family. The cDNA of SEQ ID NO: 19 contains the open reading frame (position 131 to 1120 nucleotide) which encodes the polypeptide consisting of 329 amino acids shown in SEQ ID NO: 20.

Thus, a polypeptide and polynucleotide herein described can be predicted to have biological functions and properties similar to those of the homologous polypeptides and polynucleotides, and therefore usefulness of a polypeptide and polynucleotide herein described is obvious to those skilled in the art.

Polypeptides herein described include polypeptides comprising an amino acid sequence that is at least 87% identical, preferably at least 90% identical, more preferably at least 95% (for example, 97% to 99%) identical to the amino acid sequence of SEQ ID NO: 2 or 20 in the entire sequence. Identity of amino acid sequence can be determined with, for example, the BLAST algorithm of Karlin and Altschul (Proc. Natl. Acad. Sci. USA 90: 5873-5877, 1993). Based on this algorithm, the program, BLASTX, has been developed (Altschul et al. J. Mol. Biol. 215: 403-410, 1990). When amino acid sequences are analyzed by BLASTX, the parameters are set, for example, as follows: score= 50; and wordlength= 3. When BLAST and the Gapped BLAST program are used for the analysis, the default parameters are used in each program. The specific techniques used in these analysis methods are already known (http://www.ncbi.nlm.nih.gov.). Such polypeptides can be prepared from a polynucleotide that comprises the nucleotide sequence highly homologous to the nucleotide sequence of SEQ ID NO: 1 or 19, which has been isolated using the hybridization technique, gene amplification technique, or technique of mutagenesis described below.

It is preferred that polypeptides herein described exhibit at least one of biological activities of the polypeptide comprising the amino acid sequence of SEQ ID NO: 2 or 20. Such biological activities include receptor activities of G protein-coupled receptors, such as ligand-binding activity, ; change of extracellular pH resulting from the receptor activation, change of intracellular calcium concentration, change of intracellular CAMP concentration, and change of adenylate cyclase concentration. A ligand which binds to a polypeptide herein described includes, for example, but is not limited to, histamine.

Polypeptides herein described may be in the form of a "mature" protein, or may be also a part of a larger protein, such as fusion proteins. Polypeptides herein described may contain secretory sequences, namely leader sequences; prosequences; sequences useful for purification, such as multiple histidine residues; and additive sequences to secure the stability during recombinant production.

### <Polypeptide fragments>

Also provided are fragments of polypeptides herein described. These fragments are polypeptides having amino acid sequences which are partly, but not entirely, identical to the above polypeptides herein described. Similar to polypeptides herein described, fragments may be "freestanding" (may exist itself independently) or contained in a larger polypeptide. Specifically, a fragment may constitute a portion or region of a larger polypeptide, most preferably a consecutive region of a larger polypeptide. Examples of representative polypeptide fragments herein described are, by rough estimation, fragments of amino acids at position 1 to 20, 21 to 40, 41 to 60, 61 to 80, and 81 to 100, and the fragment covering amino acids at position 101 to the polypeptide end. Herein, "rough estimation" means that one amino acid, or two, three, four, five, or several amino acids may be added or deleted at either or both ends of each region described above.

Examples of preferred fragments include truncation polypeptides, having amino acid sequences lacking a series of amino acid residues including either the amino terminus or carboxyl terminus, or two series of amino acid residues, one including the amino terminus and the other including the carboxyl terminus. Furthermore, fragments featured by structural or functional characteristics are also preferable, which include those having α-helix and α-helix forming regions, β-sheet and β-sheet forming regions, turn and turn forming regions, coil and coil forming regions, hydrophilic regions, hydrophobic regions, α-amphipathic regions, β-amphipathic regions, variable regions, surface forming regions, substrate-binding regions, and high antigenicity index region. Biologically active fragments are also preferred. Biologically active fragments mediate the activities of the polypeptides , which fragments include those having similar or improved activities, or reduced undesirable activities. For example, fragments having the activity to transduce signals into cells via binding of a ligand, and furthermore, fragments having antigenicity or immunogenicity in animals, especially humans are included.

These polypeptide fragments preferably retain the biological activities of the polypeptides, which activity includes antigenicity. Mutants of specific sequences or fragments are also provided. Preferred mutants are those which are different from the subject polypeptide, due to replacement with conservative amino acids, namely, those in which residue (s) is (are) substituted with other residue (s) having similar properties. Typical substitutions are those between Ala, Val, Leu, and Ile; Ser and Thr; acidic residues Asp and Glu, Asn, and Gln; basic residues Lys and Arg; or aromatic residues Phe and Tyr. Particularly preferable mutants are those in which several, 5 to 10, 1 to 5, or 1 to 2 amino acids are substituted, deleted, or added in any combination. Alternatively, fragments which bind to ligands without transducing signals into cells may be also useful as competitive inhibitors for the polypeptides herein described. Fragments herein described comprise 8 or more amino acid residues, preferably 12 or more (for example, 15 or more) amino acid residues.

Polypeptides of this invention may be produced by any appropriate method. Such polypeptides include isolated naturally-occurring polypeptides, and polypeptides which are produced by gene recombination, synthesis, or by a combination thereof. Procedures for producing these polypeptides are well known in the art. Recombinant polypeptides may be prepared, for example, by transferring a vector, wherein the polynucleotide herein described is inserted, into an appropriate host cell, and purifying the polypeptide expressed within the resulting transformant. On the other hand, naturally occurring polypeptides can be prepared, for example, using affinity columns, wherein antibodies against the polypeptide herein described (described below) are immobilized (Current Protocols in Molecular Biology, edit. Ausubel et al. (1987) Publish. John Wiley & Sons, Section 16.1-16.19). Antibodies for affinity purification may be either polyclonal or monoclonal antibodies. The polypeptides herein described may be also prepared by the *in vitro* translation method (see, for example, "On the fidelity of mRNA translation in the nuclease-treated rabbit reticulocyte lysate system." Dasso, M. C. and Jackson, R. J. (1989) NAR 17: 3129-3144), and such.

### <Polynucleotides>

Also provided are polynucleotides encoding the polypeptides herein described. The polynucleotides of this invention include: those encoding polypeptides comprising the amino acid sequence of SEQ ID NO: 2 or 20; those comprising the coding region of the nucleotide sequence of SEQ ID NO: 1 or 19; and those comprising different nucleotide sequences from that of SEQ ID NO: 1 or 19 due to the degeneracy of genetic codes but still encoding polypeptides comprising the amino acid sequence of SEQ ID NO: 2 or 20.

Furthermore, the polynucleotides herein described include those comprising nucleotide sequences having an identity of at least 80%, preferably at least 90%, more preferably at least 95%, and further more preferably at least 97% (for example, 98% to 99%) to the nucleotide sequence of SEQ ID NO: 1 or 19 in its entire length. Identity of the nucleotide sequences can be determined, for example, using the BLAST algorithm by Karlin and Altschul (Proc. Natl. Acad. Sci. USA 90: 5873-5877 (1993)). Based on this algorithm, an algorithm called BLASTN has been developed (Altschul et al. J. Mol. Biol. 215: 403-410 (1990)). When analyzing a nucleotide sequence using the BLASTN program, parameters are set, for example, score = 100 and wordlength = 12. When using both BLAST and Gapped BLAST programs, default parameters of each program are used. Specific techniques of these analytical methods are well known in the art (http://www.ncbi.nlm.nih.gov.). The polynucleotides herein described also include polynucleotides having a nucleotide sequences complementary to those of the above-described polynucleotides.

The polynucleotides herein described can be obtained, for example, from cDNA libraries induced from intracellular mRNAs (e.g., derived from placental cells) by standard cloning and screening methods. Moreover, the polynucleotides herein described can be obtained from natural sources, such as genomic libraries, and also can be synthesized using commercially available techniques known in the art.

Polynucleotides comprising nucleotide sequences significantly homologous to the nucleotide sequences of SEQ ID NO: 1 or 19 can be prepared using, for example, hybridization techniques (Current Protocols in Molecular Biology, edit. Ausubel et al. (1987) Publish. John Wiley & Sons Section 6.3-6.4) and the gene amplification technique (PCR) (Current Protocols in Molecular Biology, edit. Ausubel et al. (1987) Publish. John Wiley & Sons Section 6.1-6.4). That is, based on the DNA sequences encoding PLACE6002312 proteins (SEQ ID NO: 1 or 19) or portions thereof, using hybridization techniques, DNAs highly homologous to these polynucleotides can be isolated from DNA samples derived from the same or different species of organisms. Moreover, DNA fragments highly homologous to the DNA sequences encoding PLACE6002312 proteins can be isolated using the gene amplification technique by designing primers based on portions of the DNA sequences encoding PLACE6002312 proteins (SEQ ID NO: 1 or 19). Therefore, also included are polynucleotides hybridizing under stringent conditions to the polynucleotides comprising the nucleotide sequence of SEQ ID NO: 1 or 19.

As used herein, the term "stringent condition" refers to a condition under which two sequences hybridize with each other if they share at least 80%, preferably at least 90%, more preferably at least 95%, still more preferably at least 97% to 99% identity. An exemplary stringent condition includes incubation of a filter in a solution containing 5x SSC (150 mM NaCl, 15mM trisodium citrate), 50 mM sodium phosphate (pH7.6), 5x Denhardt's solution, 10% dextran sulfuric acid, and 20 µg/ml denatured and sheared salmon sperm DNA at 65°C overnight and subsequent washing in 0.1x SSC at about 65°C.

A polynucleotide that comprises a nucleotide sequence significantly homologous to the nucleotide sequence of SEQ ID NO: 1 or 19 can also be prepared using a method for introducing mutations into the nucleotide sequence of SEQ ID NO: 1 or 19 (for example, site-directed mutagenesis (Current Protocols in Molecular Biology edit. Ausubel et al., (1987) Publish. John Wiley & Sons, Section 8.1 to 8.5)). Such a polynucleotide can also be produced by spontaneous mutations. Also included are polynucleotides encoding polypeptides comprising the amino acid sequence of SEQ ID NO: 2 or 20, in which one or more amino acids have been substituted, deleted, inserted, and/or added.

There is no limitation on the number of amino acid mutations and amino acid mutation site in such polypeptides, as long as the polypeptides retain their original activity. The number of mutations is typically 30 amino acids or less, preferably 10 amino acids or less, more preferably 5 amino acids or less (for example, 3 amino acids or less). Amino acid residues to be used for substitution are preferably those having similar properties of amino acid residues to be substituted. For example, Ala, Val, Leu, Ile, Pro, Met, Phe, and Trp are all classified as non-polar amino acids, and are considered to have similar properties to each other. Further, examples of uncharged amino acids are Gly, Ser, Thr, Cys, Tyr, Asn, and Gln. Moreover, examples of acidic amino acids are Asp and Glu, and those of basic amino acids are Lys, Arg, and His.

Polynucleotides herein described that are identical or substantially identical to the nucleotide sequence contained in SEQ ID NO: 1 or 19 or the fragment thereof can be used as a probe for hybridization of cDNA and genomic DNA to isolate full-length cDNA and genomic clone encoding PLACE6002312 polypeptide and to isolate cDNAs and genomic clones for other genes comprising a sequence highly similar to the PLACE6002312 gene (including genes encoding homologs and orthologs derived from species other than human and mouse). The nucleotide sequence of such a probe is typically 80%, preferably 90%, more preferably 95% identical to the nucleotide sequence of the subject. The probe typically comprises 15 or more nucleotides, preferably 30 or more nucleotides, and may comprise 50 or more nucleotides. A particularly preferred probe consists of 30 to 50 nucleotides.

Polynucleotides used for recombinant production of the polypeptide herein described include the coding sequences of the mature polypeptide or fragments thereof alone; and coding sequences of the mature polypeptide or fragments thereof in the same reading frame with other coding sequences (for example, leader or secretory sequences; pre-, pro-, or preproprotein sequences; or sequence encoding other fusion peptide portions). For example, a marker sequence that facilitates purification of the fusion polypeptide may be encoded in the same reading frame. Also included are specific marker sequences, such as the hexahistidine peptide or Myc tag provided by the pcDNA3.1/Myc-His vector (Invitrogen), which is described in the literature (Gentz et al., Proc. Natl. Acad, Sci. USA (1989) 86: 821-824). Further, this polynucleotide may comprise a 5'- and 3'-noncoding sequence, for example, transcribed but non-translated sequences; splicing and polyadenylation signals; ribosome-binding sites; and mRNA stabilization sequences.

Polynucleotides and polypeptides herein described can be used, for example, as reagents and materials for research to discover therapeutic agents and diagnostic agents for animal and human diseases, as described below.

### <Polypeptide expression system>

Also provided is a vector containing a polynucleotide herein described, host cells genetically manipulated using such a vector, and methods for producing polypeptides herein described using the recombinant technique. A cell-free translation system can also be used to produce such polypeptides from RNAs derived from a DNA construct herein described.

To produce recombinant cells, host cells are generally manipulated for introduction of an expression system for a polynucleotide herein described. A polynucleotide can be introduced into host cells using one of the methods described in various standard laboratory manuals such as Davis et al., BASIC METHODS IN MOLECULAR BIOLOGY (1986) and Sambrook et al. , MOLECULAR CLONING: A LABORATORY MANUAL, 2nd Ed. , Cold Spring Harbor Laboratory Press, Cold Spring Harbor, N.Y. (1989), including, for example, transfection using calcium phosphate, DEAE-dextran-mediated transfection, transvection, microinjection, cationic lipid-mediated transfection, electroporation, transformation, scrapeloading, ballistic introduction, and infection.

Representative examples of the preferred host cells include bacterial cells (e.g., Streptococcus, Staphylococcus, E. coli, Streptomyces, Bacilus subtilis), fungal cells (e.g., yeast, Aspergillus), insect cells (e.g., Drosophila S2, Spodoptera SF9), animal cells (e.g., CHO, COS, HeLa, C127, 3T3, BHK, HEK293, Bowes melanoma cells), and plant cells.

Various expression systems are available. Such expression systems include, in particular, chromosomal systems, episomal systems, and viral systems. Specific examples of expression systems include, for example, vectors derived from bacterial plasmid, bacteriophage, transposon, yeast episome, insertion element, yeast chromosomal element, and virus (e.g., baculovirus, papovavirus such as SV40, vaccinia virus, adenovirus, fowl pox virus, pseudorabies virus, retrovirus), and vectors derived from combinations of the vectors described above, for example, vectors that comprise genetic elements derived from plasmid and bacteriophage, such as cosmid and phagemid. These expression systems may contain not only sequences essential for the expression but also regulatory sequences involved in the regulation of expression. In general, any systems and vectors can be used as long as they are suitable to maintain, amplify, and express a polynucleotide for the production of the corresponding polypeptide in a host. An appropriate nucleotide sequence can be introduced into an expression system by using any one of known routine techniques as described by Sambrook et al., MOLECULAR CLONING: A LABORATORY MANUAL (*supra*).

An appropriate secretion signal may be introduced into a polypeptide of interest so that the translated protein can be secreted into the endoplasmic reticulum, cell periphery or extracellular environment. Such a signal may be an endogenous or exogenous signal for the polypeptide of interest.

When polypeptides herein described are to be expressed for using in screening assays, it is preferable to produce polypeptides onto the cell surface. In such cases, cells are harvested prior to using them in screening assay. When polypeptides herein described are secreted into culture medium, the medium is collected to purify the polypeptides. When polypeptides are produced intracellulary, the polypeptides should be recovered after the cells are lysed.

Polypeptides herein described can be recovered and purified from culture of recombinant cells using a known method such as ammonium sulfate precipitation or ethanol precipitation, acid-based extraction, anion or cation exchange chromatography, phosphocellulose chromatography, hydrophobic-interaction chromatography, affinity chromatography, hydroxyapatite chromatography, and lectin chromatography. High performance liquid chromatography is most preferably used in the purification. If the polypeptides are denatured during isolation and/or purification, their active conformation can be restored using a known technique for protein refolding.

### <Diagnostic assay>

Also provided is the use of polynucleotides herein described as diagnostic agents. The detection of mutations in the genes encoding the polypeptides herein described which are correlated with functional disorders, is expected to be a useful means to diagnose a disease caused by underexpression, overexpression, or altered expression of the gene or to assess the disease susceptibility. Subjects who have mutations in the genes encoding the polypeptides herein described can be detected at the DNA level, RNA level, or protein level by using various techniques.

Polynucleotides or polypeptides to be diagnosed can be obtained from subject's cells, for example, blood, urine, saliva, and samples of tissue biopsy or autopsy. For the detection, genomic DNA may be used directly as a polynucleotide to be diagnosed. Alternatively, the genomic DNA as a polynucleotide to be diagnosed may be amplified enzymatically by PCR or another amplification method prior to the analysis. RNA or cDNA can also be used as a polynucleotide to be diagnosed in a similar way. The presence of deletion and insertion mutations can be detected based on changes in the size of the amplified product as compared with the normal genotype. Point mutations can be identified through hybridizing an amplified DNA with a labeled polynucleotide herein described. A perfectly matched sequence can be distinguished from a mismatched double-stranded sequence by digesting with RNase or based on the difference in the melting temperature between the two. Furthermore, variations in DNA sequence can be detected based on the difference of electrophoretic mobility in a gel with or without denaturant between DNA fragments or by direct DNA sequencing (see, for example, Myers et al., Science (1985) 230: 1242). Sequence variations at particular positions can be detected by nuclease protection assay (for example, RNase protection and S1 protection) or chemical cleavage (see Cotton et al., Proc. Natl. Acad. Sci. USA (1985) 85: 4397-4401).

In another embodiment, for example, an array of oligonucleotide probes containing the polynucleotides herein described or fragments thereof may be constructed to achieve efficient screening for gene mutations. The array technology has been established, has general applicabilities, and has been used to solve various problems in the molecular genetics, including gene expression, genetic linkage, and genetic mutability (see, for example, M. Chee et al., Science, Vol.274, pp. 610-613 (1996)).

The diagnostic assay which comprises the step of detecting mutations in the genes encoding the polypeptides herein described according to above-described method, provides a method for diagnosing or determining susceptibility for infection, such as bacterial infection, fungal infection, protozoiasis, and viral infection including HIV-1 or HIV-2 infection in particular, pain, cancer, diabetes, obesity, loss of appetite, hyperphagia, asthma, Parkinson's disease, acute heart failure, hypotension, hypertension, retention of urine, osteoporosis, stenocardia, myocardial infarction, ulcer, allergy, benign prostatic hypertrophy, and mental and neurologic disorders, including anxiety, schizophrenia, manic-depressive psychosis, delirium, dementia, severe mental deficiencies, such as Huntington's disease or Gilles de la .Tourette's syndrome, and dyskinesia.

In addition, diagnosis can be made for the diseases as described above using a method for detecting extraordinarily low or high levels of expression (including expression at mRNA level and at protein level) of the genes encoding the polypeptides herein described in samples derived from subjects. A decrease or increase of the expression level can be determined at RNA level by any one of polynucleotide quantitation methods known in the art, including, for example, PCR, RT-PCR, RNase protection, Northern blotting, and other hybridization methods.

Assay methods for measuring amounts of proteins, such as the polypeptides herein described, in a sample obtained from a host are well known to those skilled in the art. Such assay methods include radioimmunoassay, competitive binding assay, Western blot analysis, ELISA, etc.

It is also provided kits for diagnosing the diseases as described above or estimating the susceptibility to the diseases. Such a kit comprises: (a) a polynucleotide herein described (preferably, a polynucleotide comprising the nucleotide sequence of SEQ ID NO: 1 or 19, a fragment thereof, or a polynucleotide that has the complementary nucleotide sequence to the sequence); (b) a polypeptide herein described (preferably, the polypeptide of SEQ ID NO: 2 or 20 or a fragment thereof); or (c) an antibody against a polypeptide herein described (preferably, the polypeptide of SEQ ID NO: 2 or 20). One skilled in the art would appreciate that in such a kit (a), (b), or (c) is an essential component.

### <Chromosomal assay>

The nucleotide sequences herein described can also be used in precise mapping on the human chromosome. The mapping is performed as the first step of importance to correlate the nucleotide sequences herein described with a gene-associated disease. Thus, the physical location of a sequence on the chromosome can be correlated with data of the genetic map. Such data can be found in, for example, V. McKusick, Mendelian Inheritance in Man, available online from Johns Hopkins University Welch Medical Library. Then, the relationship between a gene mapped in an identical chromosomal region and a disease can be identified by linkage analysis (co-heritability of genes physically adjacent to each other).

It is possible to determine the difference in cDNA or genomic sequence between patients and normal subjects. When some or all patients are found to carry a mutation but no normal subject has the mutation, the mutation can be a cause of the disease.

### <Antibodies>

A polypeptide herein described or its fragment, or analogs thereof, or a cell that expresses them can be used as an immunogen for producing antibodies binding to the polypeptide herein described. The antibodies are preferably immunospecific to a polypeptide herein described. The term "immunospecific" means that the antibody has substantially higher affinity to the polypeptides of the present invention than to other related polypeptides known in the art.

Antibodies which bind to a polypeptide herein described can be prepared by administering a fragment, analog, or cell containing the polypeptide or epitope to an animal (preferably, other than human) using a conventional protocol. Any appropriate technique using continuously-cultured cell system that produces antibodies can be used to prepare monoclonal antibodies. For example, such techniques include hybridoma technique (Kohler, G. and Milstein, C., Nature (1975) 256: 495-497), trioma technique, human B cell hybridoma technique (Kozbor et al . , Immunology Today (1983) 4: 72), and EBV-hybridoma technique (Cole et al. , MONOCLONAL ANTIBODIES AND CANCERTHERAPY, pp. 77-96, Alan R. Liss, Inc. , 1985).

Transgenic mice or other non-human animals including other non-human mammals can be used to express humanized antibodies against a polypeptide herein described. A clone expressing the polypeptide can be isolated and identified using the above-mentioned antibody, and the polypeptide can be purified by affinity chromatography using the antibody. An antibody binding to a polypeptide herein described may be used for treating, in particular, infection, such as bacterial infection, fungal infection, protozoiasis, and viral infection including HIV-1 or HIV-2 infection in particular, pain, cancer, diabetes, obesity, loss of appetite, hyperphagia, asthma, Parkinson's disease, acute heart failure, hypotension, hypertension, retention of urine, osteoporosis, stenocardia, myocardial infarction, ulcer, allergy, benign prostatic hypertrophy, and mental and neurologic disorders, including anxiety, schizophrenia, manic-depressive psychosis, delirium, dementia, severe mental deficiencies, such as Huntington's disease or Gilles de la Tourette's syndrome, and dyskinesia.

### <Vaccine>

Also provided is a method for potentiating the immune response in mammals. The method comprises the step of inoculating a sufficient amount of a polypeptide herein described or a fragment thereof for producing antibodies and/or causing T-cell immune response for protecting the animals against, in particular, infection, such as bacterial infection, fungal infection, protozoiasis, and viral infection including HIV-1 or HIV-2 infection in particular, pain, cancer, diabetes, obesity, loss of appetite, hyperphagia, asthma, Parkinson's disease, acute heart failure, hypotension, hypertension, retention of urine, osteoporosis, stenocardia, myocardial infarction, ulcer, allergy, benign prostatic hypertrophy, and mental and neurologic disorders, including anxiety, schizophrenia, manic-depressive psychosis, delirium, dementia, severe mental deficiencies, such as Huntington's disease or Gilles de la Tourette's syndrome, and dyskinesia.

Also provided is a method for potentiating the immune response in mammals, which comprises supplying a polypeptide herein described *in vivo* via a vector that directs the expression of a polynucleotide herein described to potentiate the immune response so as to produce antibodies that prevent diseases in mammals.

Also provided are immunological/vaccine preparations (compositions) to potentate the immune response to a polypeptide herein described in a mammalian host when introduced into the mammalian host. This composition contains a polypeptide or polynucleotide herein described The vaccine preparations may further contain appropriate carriers. The PLACE6002312 polypeptide can be decomposed in the stomach, and therefore preferably administered parenterally (including subcutaneous, intramuscular, intravenous, and intracutaneous injection). Preparations suitable for parenteral administration include aseptic aqueous or non-aqueous injection solution that can contain an antioxidant, a buffer, a bacteriostat, and a solute that makes the preparation isotonic to subject's blood, and aseptic aqueous or non-aqueous suspension that can contain an emulsifier or a thickening agent. Such preparations can be provided in single-dose containers or multi-dose containers (for example, closed ampules and vials), or alternatively stored as a freeze-dried form to which an aseptic liquid carrier is added immediately before use. The vaccine preparations may comprise an adjuvant system to enhance the immunogenicity of this preparation, for example, an oil-in-water adjuvant system and other adjuvant systems known in the art. The dose of vaccine depends on its specific activity, and can be determined simply by a routine experimental procedure.

### <Screening assay>

The polypeptides herein described can be used in a screening method for a compound (i.e., agonist) that activates the polypeptides or a compound (i.e., antagonist, also referred to as inhibitor that inhibits their activities. The polypeptides herein described can be used, for example, to assess binding of low-molecular-weight substrates and ligands present in cells, cell-free preparations, libraries of chemical substances, or mixtures of natural products. Such substrates and ligands may be natural substrates or ligands, or structural or functional mimics (see Coligan et al. , Current Protocols in Immunology 1 (2) : Chapter 5(1991)).

The polypeptides herein described are involved in various biological functions including many pathologies. Thus, on one hand, it is desired to screen for compounds and drugs that can activate the polypeptides herein described, but on the other desirable is to screen for those that can inhibit the function of the polypeptides herein described. In general, agonists and antagonists are used for treating and preventing symptoms for infection, such as bacterial infection, fungal infection, protozoiasis, and viral infection including HIV-1 or HIV-2 infection in particular, pain, cancer, diabetes, obesity, loss of appetite, hyperphagia, asthma, Parkinson's disease, acute heart failure, hypotension, hypertension, retention of urine, osteoporosis, stenocardia, myocardial infarction, ulcer, allergy, benign prostatic hypertrophy, and mental and neurologic disorders, including anxiety, schizophrenia, manic-depressive psychosis, delirium, dementia, severe mental deficiencies, such as Huntington's disease or Gilles de la Tourette's syndrome, dyskinesia, etc.

Typically, such a screening method is performed using appropriate cells expressing a polypeptide herein described on their surface. Such cells include cells derived from mammals , yeasts, Drosophila, and E. coli. Then, a test compound is contacted with cells expressing a receptor (or cell membrane containing the expressed polypeptide herein described) to observe their binding, or activation or inhibition of functional response.

An embodiment of screening techniques encompasses the use of cells expressing a receptor herein described (for example, transfected CHO cell) in an assay system for the change of extracellular pH or change of intracellular calcium level, which results from the activation of the receptor. In this method, a compound can be contacted with cells expressing a polypeptide herein described. Then, responses mediated with a second messenger (for example, signal transduction, pH change, or change of calcium level) are detected to determine whether a candidate compound activates or inhibits the receptor.

Another method of screening for receptor inhibitors comprises determining inhibition or activation of the accumulation of receptor-mediated CAMP and/or adenylate cyclase. This method comprises transfecting eukaryotic cells with a polypeptide herein described and allowing the expression of the receptor on the cell surface. Then, the amount of accumulated CAMP is determined after the cells are contacted with a candidate antagonist in the presence of the receptor herein described. When the binding of the candidate antagonist to the receptor results in inhibition of receptor binding, the level of activity of receptor-mediated cAMP or adenylate cyclase will be decreased or increased.

Furthermore, a polynucleotide (for example, cDNA) or a polypeptide herein described or an antibody against the polypeptide can be used to construct an assay system for determining the action of a compound on the intracellular expression (production of mRNA or protein) of a gene encoding the polypeptide herein described. For example, a monoclonal or polyclonal antibody can be used to establish ELISA for determining the secreted level or the cell binding level of a polypeptide herein described using a standard method known in the art. The ELISA method can be used to explore substances suppressing or enhancing the production of a polypeptide herein described in appropriately manipulated cells or tissues. The standard method of screening assay is well known in the art.

Exemplary potential antagonists of a polypeptide herein described include antibodies, and in some cases, oligonucleotides or proteins closely related to a ligand (for example, a fragment of a ligand), and small molecules which bind to a polypeptide herein described but do not induce the response (thus, inhibit the receptor activity).

Also provided is a screening kit to identify agonists, antagonists, ligands, receptors, substrates, enzymes, or such of polypeptides herein described, or compounds that decrease or increase the production of polypeptides herein described. Such a kit comprises: (a) a polypeptide herein described (preferably, the polypeptide of SEQ ID NO: 2 or 20) ; (b) recombinant cells expressing the polypeptide herein described (preferably, the polypeptide of SEQ ID NO: 2 or 20) or cell membrane thereof; (c) an antibody against the polypeptide herein described (preferably, the polypeptide of SEQ ID NO: 2 or 20). One skilled in the art would appreciate that in such a kit (a), or (c) is an essential component.

### <Preventive and therapeutic methods>

Also provided is a method for treating abnormal conditions, which associate with both overexpression and underexpression of activity of polypeptides herein described. Such conditions include infection, such as bacterial infection, fungal infection, protozoiasis, and viral infection including HIV-1 or HIV-2 infection in particular, pain, cancer, diabetes, obesity, loss of appetite, hyperphagia, asthma, Parkinson's disease, acute heart failure, hypotension, hypertension, retention of urine, osteoporosis, stenocardia, myocardial infarction, ulcer, allergy, benign prostatic hypertrophy, and mental and neurologic disorders, including anxiety, schizophrenia, manic-depressive psychosis, delirium, dementia, severe mental deficiencies, such as Huntington's disease or Gilles de la Tourette's syndrome, and dyskinesia. Some approaches are available when a polypeptide herein described is activated excessively. One approach comprises administering an antagonist as described above in combination with pharmaceutically acceptable carriers to a patient in an amount effective to inhibit the activity, thereby inhibiting the binding of a ligand to the polypeptide herein described or suppressing the second signal to ameriolate the abnormal conditions. Such antagonists include a soluble polypeptide of the present invention capable of binding to a ligand competitively with an endogenous polypeptide herein described. A typical example of such competitive substances is a fragment of polypeptides herein described.

Another approach comprises suppressing the expression of a gene encoding an endogenous polypeptide herein described using an expression inhibition method. Such a known technique requires the use of an antisense sequence that is produced in vivo or separately administered. See, for example, O'Connor, J. Neurochem. (1991) 56: 560, in Oligodeoxynucleotides as Antisense Inhibitors of Gene Expression, CRC Press, Boca Raton, FL (1988. Alternatively, it is possible to administer an oligonucleotide that forms a triple helix with this gene. See, for example, Lee et al., Nucleic Acids Res (1979) 6: 3073; Cooney et al., Science (1988) 241: 456; and Dervan et al., Science (1991) 251: 1360. Such oligomers themselves may be administered, or related oligomers may be expressed *in vivo.*

A number of approaches can be used to treat abnormal conditions resulting from the underexpression of the activity of a polypeptide herein described. One approach comprises administering to a patient a therapeutically effective amount of a compound (namely, an agonist as described above) that activates a polypeptide herein described, in combination with pharmaceutically acceptable carriers, to ameriolate the abnormal conditions. Another approach is gene therapy for producing a polypeptide herein described endogenously in related cells of a patient. For example, a polynucleotide herein described is engineered genetically to express it in a replication-deficient retroviral vector as described above. Then, the retroviral expression construct is isolated and introduced into packaging cells transformed with a retroviral plasmid vector that contains RNA encoding the polypeptide herein described. The resulting packaging cells produce infectious virus particles containing the gene of interest. These virus-producing cells are introduced into the patient to treat cells in vivo and to express the polypeptide in vivo. For the outline of gene therapy, see Chapter 20, Gene Therapy and other Molecular Genetic-based Therapeutic Approaches (and the references cited therein) in Human Molecular Genetics, T. Strachanand A. P. Read, BIOS Scientific Publishers Ltd (1996). Another approach comprises administering a therapeutically effective amount of a polypeptide herein described in combination with appropriate pharmaceutically acceptable carriers.

A preparation and a soluble administrable form of a polypeptide, agonist, antagonist, or small molecule herein described can be formulated in combination with appropriate pharmaceutically acceptable carriers. Such a preparation contains a therapeutically effective amount of a polypeptide or compound and pharmaceutically acceptable carriers or excipients. Such carriers include, but are not limited to, saline, physiological saline, dextrose, water, glycerol, ethanol, and combinations thereof. The preparation should depend on the mode of administration, and this can be achieved using a technique known in the art.

Furthermore, it is provided a pharmaceutical package or kit that comprises one or more containers filled with one or more components of a composition herein described describe above. A polypeptide and other compounds herein described may be used alone or in combination with other compounds, for example, therapeutic compounds.

A preferred procedure for systemic administration of a pharmaceutical composition is infusion (injection), typically intravenous injection. It is also possible to use other infusion pathways, such as subcutaneous, intramuscular, or intraperitoneal injection. Alternative systemic administration means include transmucosal and transdermal administration using a penetrant, such as bile salt, fusidic acid, or another surfactant. The compounds can also be administered orally, when formulated appropriately as an enteric-coated preparation or a capsule. These compounds can be applied topically and/or localized as a dosage form, such as ointment, paste, or gel.

The range of required dose depends on the type of selected peptide, administration pathway, properties of the preparation, patient's conditions, and physician's judgment. However, an appropriate dose typically falls within the range of 0.1 to 100 µg/kg patient's weight. Since there are various compounds available and their administration pathway and efficacy differ depending on compounds, one should note that the required dose may vary within a wide range. For example, oral administration is predicted to need a higher dose as compared to administration by intravenous injection. Such dose alterations can be determined using the empirical standard optimization procedure well known in the art.

A polypeptide to be used in therapy can be produced in patient's body with the therapeutic method called "gene therapy" as described above. For example, cells derived from a patient are genetically manipulated ex vivo with a polynucleotide, such as DNA or RNA, encoding a polypeptide using, for example, a retroviral plasmid vector. Then, the cells are introduced into the patient:

### Brief Description of the Drawings

Fig. 1 shows the result obtained by BLAST search of the entire SWISS-PROT sequences using the amino acid sequence of PLACE6002312 as a "Query". The amino acid sequence of PLACE6002312 had the highest homology (27%) to HISTAMINE H2 RECEPTOR (GenBank accession No. P97292).

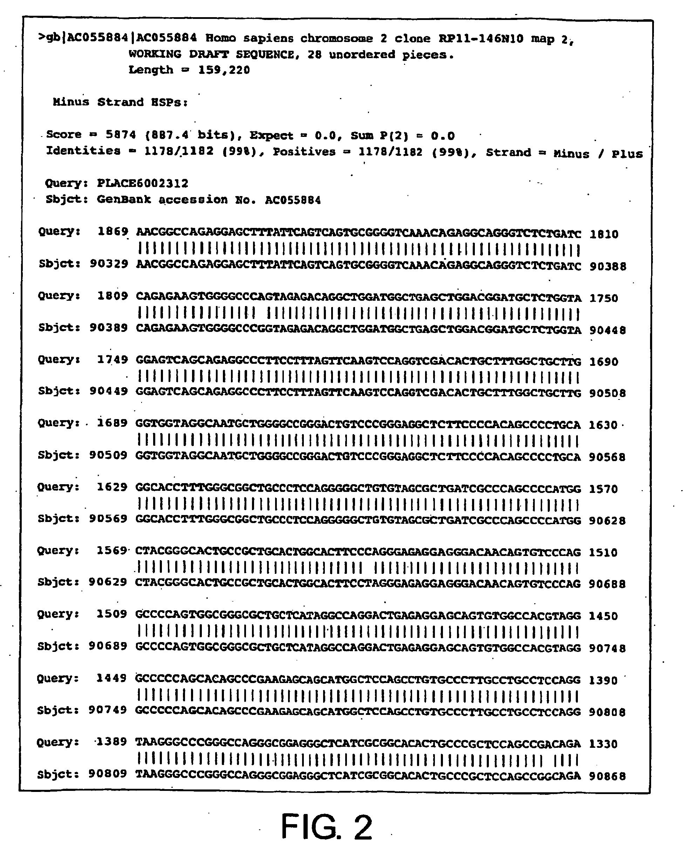

Fig. 2 shows the result obtained by BLAST search of GenBank using the nucleotide sequence of PLACE6002312 as a "Query". The nucleotide sequence of PLACE6002312 had the highest homology (99%) to a clone from the draft sequences for the human genome (GenBank accession No. AC021016, Homo sapience chromosome 2 clone RP11-378A13, WORKING DRAFT SEQUENCE, 7 unordered pieces). In addition, the fragment sequences of two splitting parts of the nucleotide sequence of PLACE6002312 were respectively homologous to different regions in a continuous genome sequence. It was presumable that non-identical nucleotide residues between the nucleotide sequences resulted from genetic polymorphisms.
Fig. 3 is continued from Fig. 2.
Fig. 4 is continued from Fig. 3.
Fig. 5 is a photograph showing an expression profile obtained by RT-PCR assay for PLACE6002312 gene. A DNA fragment of about 600 bp was amplified using samples derived from lung, heart, ovary, testis, liver, colon, and placenta.
Fig. 6 is a photograph showing an expression profile obtained by Northern blotting analysis for PLACE6002312 gene. About 1.7 kb RNA fragment was detected as PLACE6002312 mRNA in samples derived from heart, placenta, liver, kidney, pancreas, spleen, ovary, small intestine, colon, and peripheral blood leukocyte.

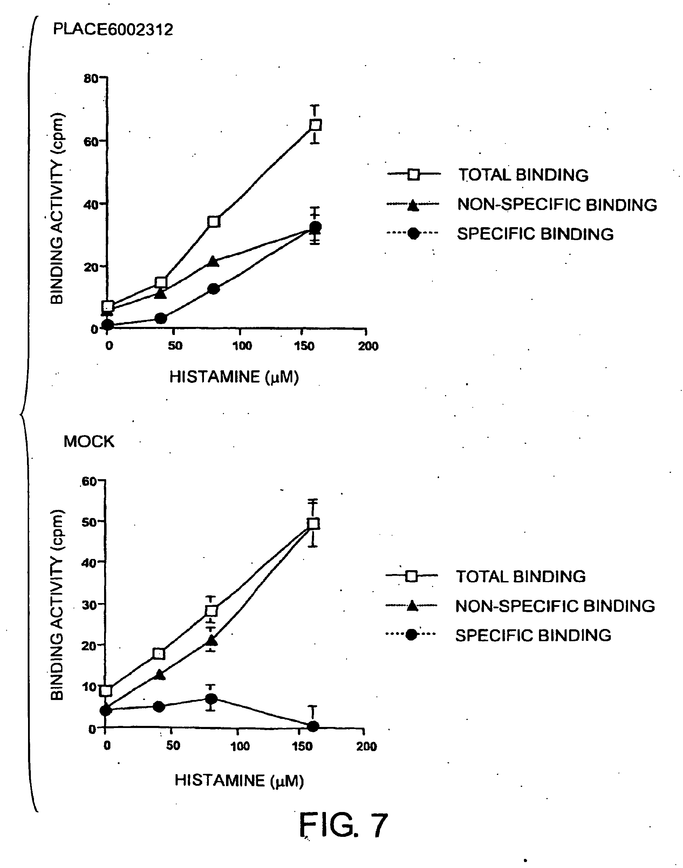

Fig. 7 shows concentration-dependent binding between PLACE6002312 polypeptide and histamine. Specific binding to histamine was measured using cultured cells and was analyzed from the difference between the total binding activity and non-specific binding activity to [³H]-histamine (each data point was determined at n= 6; the standard deviations are indicated). Specific binding to histamine was detected in HEK293 cells in which an expression vector for PLACE6002312 polypeptide had been introduced. Conversely, specific binding to histamine was undetectable in HEK293 cells in which a plasmid that does not express PLACE6002312 polypeptide had been introduced. Thus, PLACE6002312 gene was revealed to encode a polypeptide that specifically binds to histamine.
Fig. 8 shows an alignment of the amino acid sequence of PLACE6002312 and its mouse homolog. The upper part of the alignment corresponds to the sequence of PLACE6002312; the bottom part, mouse homolog.

### Best Mode for Carrying out the Invention

The present invention is illustrated in detail below with reference to Examples, but is not to be construed as being limited thereto. Unless otherwise stated, the experiments shown in these Examples can be carried out according to known methods (Maniatis, T. et al., (1982) Molecular Cloning - A Laboratory Manual, Cold Spring Harbor Laboratory, NY).

### [Example 1] Isolation of G protein-coupled receptor PLACE6002312

The G protein-coupled receptor PLACE6002312 was isolated using a cDNA library prepared from human placental total RNA (Clontech). Specifically, the total RNA sample was treated with bacterial alkaline phosphatase (BAP), and then further treated with tobacco alkaline phosphatase (TAP) to convert the CAP structure of the 5' end of full-length mRNA into a phosphate group. Using RNA ligase, the resulting mRNA was ligated with a synthetic oligo RNA linker (sequence: 5'- AGC AUC GAG UCG GCC UUG UUG GCC UAC UGG -3'/SEQ ID NO: 3). After the first strand cDNA was synthesized using the mRNA as a template and an oligo dT adapter primer (sequence: 5'-GCG GCT GAA GAC GGC CTA TGT GGC CTT TTT TTT TTT TTT TTT -3'/SEQ ID NO: 4), PCR was carried out using Gene Amp XL PCR kit (Perkin Elmer) and forward primer (5'- AGC ATC GAG TCG GCC TTG TTG -3' /SEQ ID NO: 5) and reverse primer (5'- GCG GCT GAA GAC GGC CTA TGT GGC CTT TTT TTT TTT TTT TTT -3' /SEQ ID NO: 6) under the cycling conditions of 1 cycle of 95°C for 5 min, followed by 15 cycles of 95°C for 1 min, 58°C for 1 min, and 72°C for 10 min. After the PCR amplification products were digested with the restriction enzyme SfiI, using DNA ligation kit version 1 (Takara Shuzo), the resulting fragment was ligated to the cloning vector pME18SFL3 pre-digested with the restriction enzyme DraIII. The nucleotide sequence of cloned cDNA was analyzed by the dideoxy terminator method using ABI3700 DNA Sequencer (Applied Biosystems). The determined sequence is shown in SEQ ID NO: 1.

This sequence consists of 1869 nucleotide residues, and contains an open reading frame of 990 nucleotides (SEQ ID NO: 7). The amino acid sequence (330 amino acid residues) deduced from the open reading frame is shown in SEQ ID NO: 2. The deduced amino acid sequence contains a hydrophobic domain which is estimated as seven transmembrane domains characteristic of G protein-coupled receptor. In addition, this sequence was found to contain amino acid residues characteristic of G protein-coupled receptor whose ligand can be monoamine or histamine (Leurs, I., Hoffmann, I., Wieland, I., and Timmerman, I. (2000) Trends Pharmacol. Sci. 21: 11), including the aspartic acid residue located immediately after the first transmembrane domain, DRY (or ERY) sequence located immediately after the third transmembrane domain, tryptophane residue in the fourth transmembrane domain, cysteine residue between the fourth and fifth transmembrane domains, and the WXP sequence in the sixth transmembrane domain . Thus, the gene was revealed to encode a G protein-coupled receptor.

### [Example 2] BLAST search of SWISS-PROT for the amino acid sequence of G protein-coupled receptor PLACE6002312

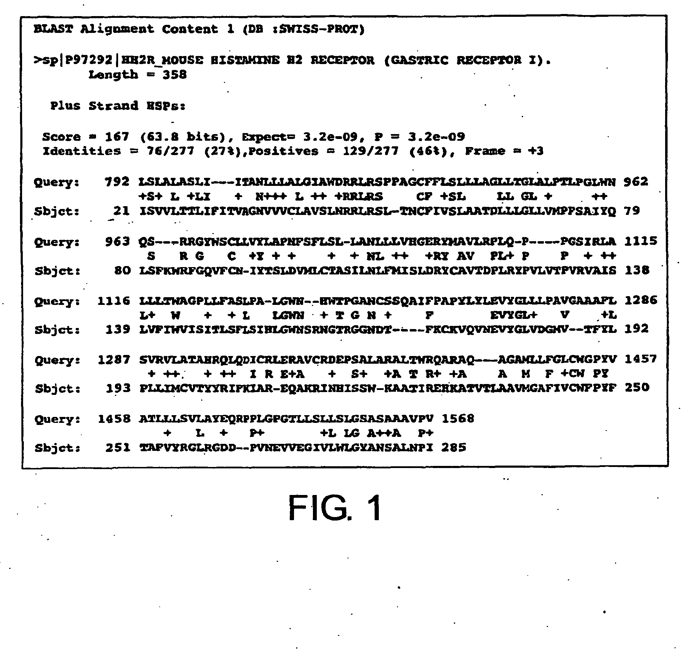

The SWISS-PROT databank was searched for the amino acid sequence of PLACE6002312 using BLAST (Basic local alignment search tool; S. F. Altschul et al., J. Mol. Biol., 215: 403-410 (1990)). The result obtained is shown in Fig. 1. No identical sequence was found, but the sequence had the highest homology (27%) to HISTAMINE H2 RECEPTOR (GenBank accession No. P97292). These findings showed that PLACE6002312 was a novel G protein-coupled receptor.

### [Example 3] BLAST search of GenBank for the gene of G protein-coupled receptor PLACE6002312

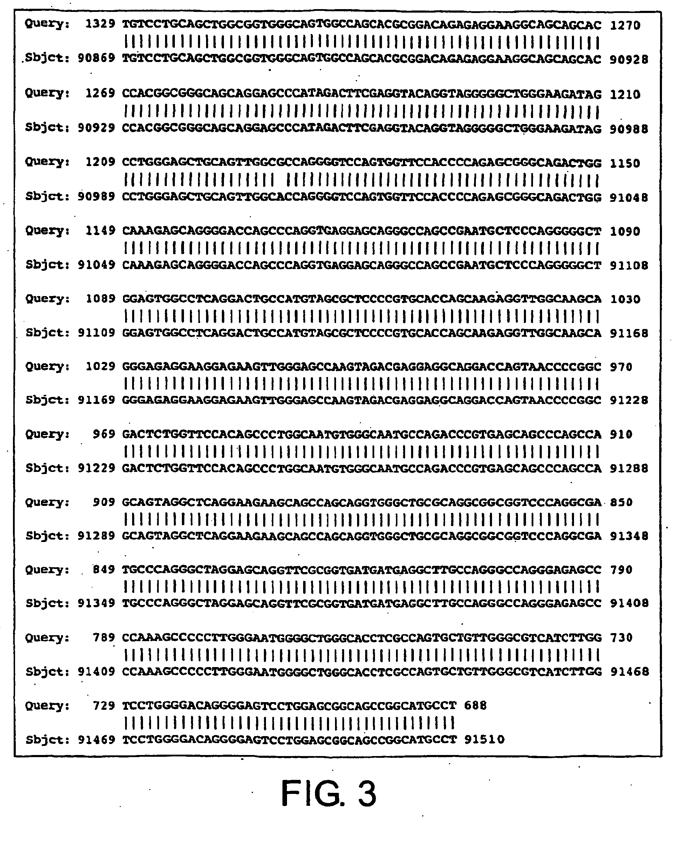

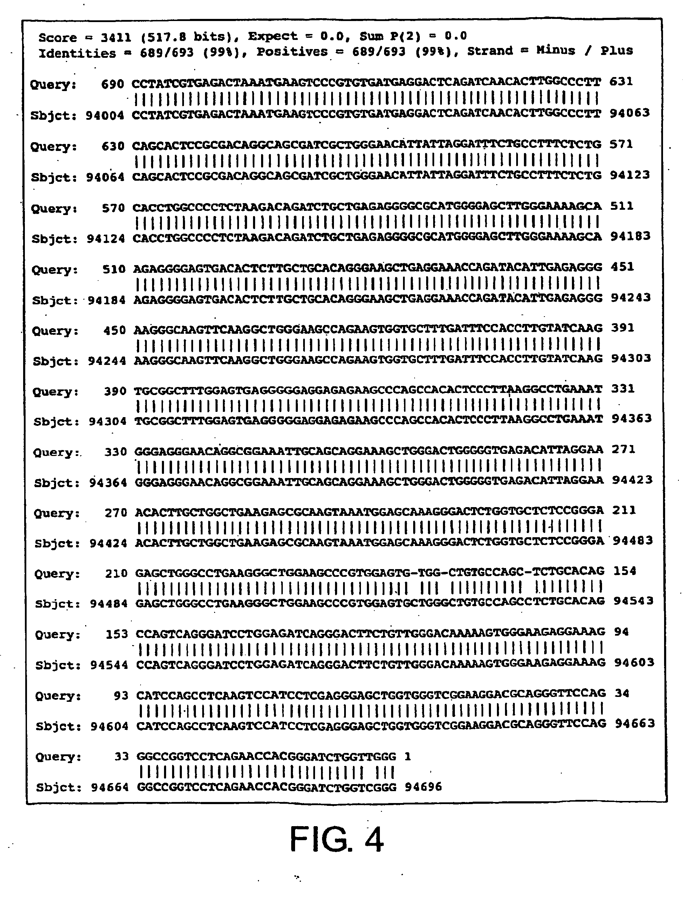

BLAST search of GenBank was carried out for the nucleotide sequence of PLACE6002312. The result obtained is shown in Figs. 2 to 4. The nucleotide sequence had the highest homology (99%) to a clone from the draft sequences for the human genome (GenBank accession No. AC021016; Homo sapience chromosome 2 clone RP11-378A13, WORKING DRAFT SEQUENCE, 7 unordered pieces). Thus, it was revealed that the gene of G protein-coupled receptor PLACE6002312 was positioned on human chromosome 2 and comprised two exons. In addition, in the nucleotide sequence of AC021016, a nucleotide sequence located 5' more upstream of a nucleotide sequence homologous to the 5'-end nucleotide sequence of PLACE6002312 was found to correspond to the transcriptional regulatory region of the PLACE6002312 gene. In addition, non-identical nucleotide residues to each other were estimated to result from genetic polymorphisms.

### [Example 4] Distribution of expression of the PLACE6002312 gene in human tissues

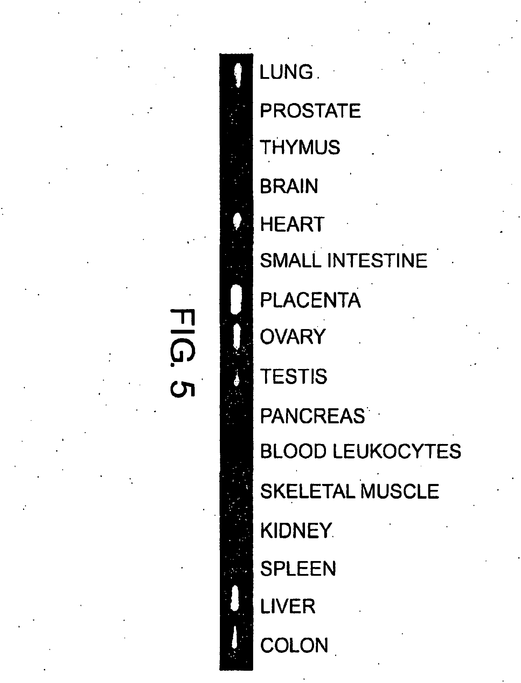

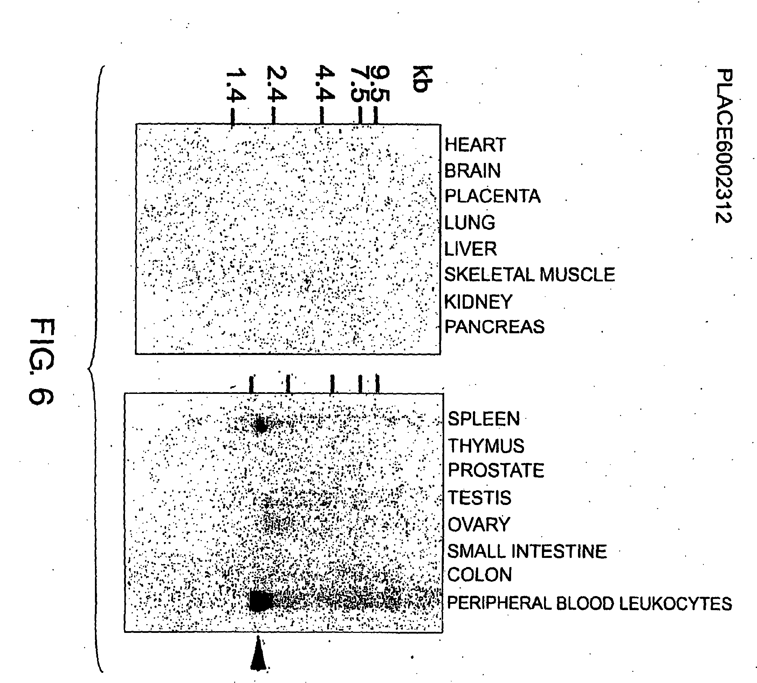

The tissue distribution of PLACE6002312 was studied using MTC panel (Clontech). The primers used were designed to flank the region that amplifies a fragment of about 600 bp in the ORF of the clone; primer 1: 5'- CAT GGC AGT CCT GAG GCC ACT CCA GC -3' (SEQ ID NO: 8) and primer 2: 5'- AGG CAC CTT TGG GCG GCT GCC CTC CA -3' (SEQ ID NO: 9). PCR was carried out using LA Taq DNA polymerase (Takara Shuzo) under the cycling conditions of 1 cycle of 94°C for 5 min, followed by 33 cycles of 98°C for 20 sec and 68°C for 5 min. As a result, the cDNA fragment of about 600 bp was amplified from cDNAs derived from lung, heart, ovary, testis, liver, colon, and in particular placenta, but such amplification was not detectable when cDNA was prepared from other organs (prostate, thymus, brain, pancreas, leukocytes, skeletal muscle, kidney, and spleen) (Fig. 5). The expression profile was also studied by Northern blotting analysis. Using DNA labeling kit BcaBEST (Takara Shuzo), a labeled probe was prepared from a 528-bp fragment (SEQ ID NO: 10) that was obtained by treating PLACE6002312 DNA with the restriction enzyme Eco47III. Northern blotting was carried out by hybridizing the probe to MTN blots (Clontech). The result showed that the PLACE6002312 gene was transcribed into mRNA of about 1.7 kb and the band corresponding to the transcript was detectable in tissues and cells, including heart, placenta, liver, kidney, pancreas, spleen, ovary, small intestine, colon, and peripheral blood leukocytes. However, the transcript was undetectable in mRNAs from other organs (brain, lung, skeletal muscle, thymus, prostate, and testis) (Fig. 6).

### [Example 5] Construction of expression plasmid for PLACE6002312

In order to maximize the expression level of the polypeptide encoded by the PLACE6002312 gene, the entire nucleotide residues of the 5'- and 3'- untranslated regions (UTRs) were removed from the cDNA, before the PLACE6002312 gene was inserted into the expression vector. Specifically, in order to obtain a DNA fragment containing only the coding region for the polypeptide from the PLACE6002312 gene, a primer pair were prepared, which consist of primer 3: 5'- GGA ATT CAT TTA GTC TCA CGA TAG GCA TG -3'/SEQ ID NO: 11 and primer 4: 5'- CGG GAT CCT CAG TGC GGG GTC AAA CAG AGG CA -3' /SEQ ID NO: 12, carrying the added restriction enzyme sites of EcoRI and BamHI, respectively. And then PCR was carried out using LA Taq DNA polymerase (Takara Shuzo), together with the PLACE6002312 DNA ligated with pME18SFL3 as a template DNA. A DNA fragment of about 1.2 kb was amplified under the cycling conditions of 1 cycle of 94°C for 5 min, followed by 30 cycles of 98°C for 20 sec and 68°C for 5 min. The fragment was treated with the restriction enzymes EcoRI and BamHI, and then was ligated, using DNA ligation kit version 1 (Takara Shuzo), to the expression vector pcDNA3.1(-) (Clontech) pre-digested with the same restriction enzymes, EcoRI and BamHI. The nucleotide sequence of the ligated DNA fragment was analyzed by the dideoxy terminator method in an ABI 377 DNA Sequencer. After the nucleotide sequence was confirmed, the constructed plasmid was named pcDNA3.1(-)-PLACE6002312.

### [Example 6] Specific binding between PLACE6002312 protein-expressing 293 cell and histamine

The ligand-binding assay can serve as a direct method of receptor pharmacology, and is applicable to high-throughput systems. The experiments described below were carried out to reveal that the polypeptide encoded by the PLACE6002312 gene has histamine-binding activity. First, human embryonic kidney 293 (HEK293) cells were plated on a 10-cm cell culture dish at the cell density of 1x 10⁶ cells/dish, and incubated in a humidified incubator under 5% CO₂ at 37°C for 24 hr. Then, 10 µg/dish of pcDNA3.1(-)-PLACE6002312 plasmid was transfected by the calcium phosphate method (Mol. Pharm. (1997) 51: 171). After the culturing was continued for 14 hr, the medium was changed with fresh D-MEM containing 10% FBS, and the cells were incubated for another 2 days. After the culturing had completed, the cells were washed with PBS(-), and then suspended in FBS-free D-MEM at the cell density of 1x 10⁶ cells/ml. The cell suspension were aliquoted (50 µl/well) into flat-bottomed 96-well plate (Sumitomo Bakelite Co.) pre-siliconized with Siliconize L-25 (Fuji Systems). 50-µl aliquots of D-MEM were added to each well, and then radio-labeled [³H]-histamine was added thereto at the final concentrations of 0, 40, 80, and 160 nM. The plate was incubated at room temperature for 1 hr. Assays for the non-specific binding of [³H]-histamine were carried out using D-MEM to which unlabeled histamine had been added previously at the final concentration of 1x 10⁻⁵ M.

After the reaction had completed, the cell suspension was filtered by vacuum filtration using 96-well filters. The white microplate with bonded GF/B filter (Packard) was used as the 96-well filter. Each well was pre-treated by filtration with 150 µl of 0.3% polyethyleneimine. Then, the wells were washed seven times with 150 µl/well of ice-cold washing buffer (50 mM Tris-Cl (pH7.4), 5 mM MgCl₂), and then the filter was dried at 47°C for about 14 hr. The radioactivity of bound [³H]-histamine was determined by adding 40 µl/well of MICROSCINT-40 (Packard) and using TOPCOUNT (Packard) . The specific binding activity to [³H]-histamine was analyzed based on the difference between the total binding activity (without unlabeled histamine) and non-specific binding activity (with unlabeled histamine). As a result, the specific binding to [³H]-histamine was detected in the HEK293 cells in which pcDNA3.1(-)-PLACE6002312 had been introduced. Conversely, the non-specific binding to [³H]-histamine was observed, while the specific binding was not in the HEK293 cells (Mock) in which pcDNA3.1(-) had been introduced (Fig. 7). Thus, PLACE6002312 was revealed to encode a polypeptide which specifically binds to histamine. Using this ligand-binding assay system, screening can be carried out to select antagonists and agonists of G protein-coupled receptor PLACE6002312.

### [Example 7] Isolation of mouse homolog cDNA of PLACE6002312

Mouse homolog cDNA of PLACE6002312 was isolated by the method of rapid amplification of cDNA ends (RACE). The template cDNA used was mouse liver-derived Marathon-Ready^{™} cDNA (Clonetech). PCR was performed using the thermostable DNA polymerase, Pfu DNA polymerase (Stratagene). Specifically, 3' primer for amplification (sequence: 5'- CAGCTCCCAGGCTATCTTCCCAGC -3'/SEQ ID NO: 13) and adapter primer 2 (sequence: 5'- ACTCACTATAGGGCTCGAGCGGC -3' /SEQ ID NO: 14) were prepared based on a particular nucleotide sequence in the open reading frame of PLACE6002312. The 3' fragment of mouse homolog cDNA of PLACE6002312 was isolated by PCR using the primers under the cycling conditions of 1 cycle of 95°C for 5 min, followed by 30 cycles of 95°C for 30 sec, 60°C for 30 sec, and 72°C for 3 min, and then 1 cycle of 72°C for 10 min. The 5' fragment of the mouse homolog cDNA of PLACE6002312 was isolated by PCR using 5' primer for amplification (sequence: 5'- GCACTCGTAGACACCTTTGGGCAGC -3'/SEQ ID NO: 15) and adapter primer 1 (sequence: 5'-CCATCCTAATACGACTCACTATAGGGC -3'/SEQ ID NO: 16), under the cycling conditions of 1 cycle of 95°C for 5 min, followed by 30 cycles of 95°C for 30 sec, 60°C for 30 sec, and 72°C for 3 min, and then 1 cycle of 72°C for 10 min. These cDNA fragments were ligated with the cloning vector pCR®2.1-TOPO. The nucleotide sequences of the cloned cDNAs were analyzed by the dideoxy terminator method in an ABI3700 DNA Sequencer (Applied Biosystems). The full-length cloning primer 1 (sequence: 5'- ATGGCCCGAAGACCTGCAAGAGTG -3'/SEQ ID NO: 17) and full-length cloning primer 2 (sequence: 5'-TGGCCAGAGATTTATTTGGTCTGGGTAGGT -3'/SEQ ID NO: 18) to clone the full-length mouse cDNA were prepared based on the nucleotide sequences of the mouse cDNAs as described above. PCR was carried out under the cycling conditions of 1 cycle of 95°C for 4 min, followed by 30 cycles of 95°C for 30 sec, 60°C for 30 sec, and 72°C for 3 min, and then 1 cycle of 72°C for 10 min to isolate the full-length cDNA for the mouse homolog of PLACE6002312. The full-length cDNA was inserted into the cloning vector pCR®2.1-TOPO, and the full-length nucleotide sequence was analyzed by the dideoxy terminator method. The determined sequence is shown in SEQ ID NO: 19.

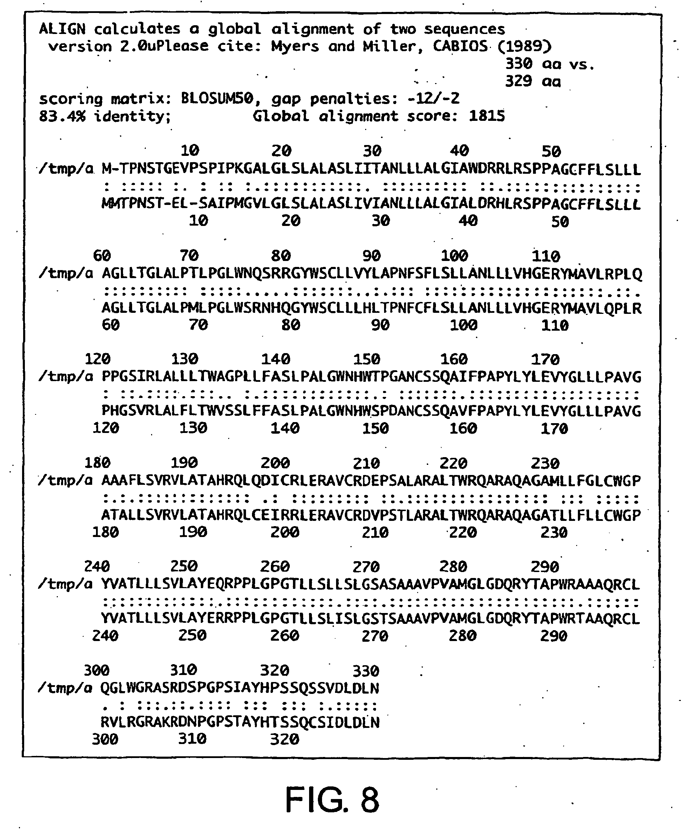

This sequence consists of 1237 nucleotides, and contains an open reading frame of 990 nucleotides. The amino acid sequence deduced from the open reading frame (329 amino acids) is shown in SEQ ID NO: 20. The deduced amino acid sequence contains hydrophobic regions which are estimated as seven transmembrane domains characteristic of G protein-coupled receptor. The sequence has 8.2% homology at nucleotide level and 82% at amino acid level to PLACE60023. An alignment of PLACE6002312 and its homologous amino acid sequence is shown in Fig. 8. These findings show that the gene is the mouse homolog of PLACE6002312.

### Industrial Applicability

It is provided novel G protein-coupled receptors that bind to histamine, polynucleotides encoding the polypeptides, vectors containing the polynucleotides, host cells containing the vectors, and a method for producing the polypeptides. Also provided is a method of screening for compounds that modify the activity of the polypeptides. Polypeptides and polynucleotides herein described, and compounds that modifies the activity of polypeptides herein described are expected to be used for developing new preventives and therapeutics for diseases in which the histamine receptors herein described are involved. In addition, it is provided a method for determining whether certain disease is caused by mutations in genes encoding polypeptides herein described and mutations in the flanking regions of the genomic sequence of the genes, and thus is expected to be used in gene diagnosis.

## Claims

1. In vitro Use of a polypeptide encoded by a polynucleotide selected from any one of (a) through (d):
(a) a polynucleotide which encodes a polypeptide comprising the amino acid sequence of SEQ ID NO: 2;
(b) a polynucleotide which comprises a coding region of the nucleotide sequence of SEQ ID NO: 1;
(c) a polynucleotide which hybridizes to a DNA comprising the nucleotide sequence of SEQ ID NO: 1 under a stringent condition;
(d) a polynucleotide which encodes a fragment of a polypeptide comprising the amino acid sequence of SEQ ID NO: 2;
as histamine-binding receptor.

## Patentansprüche

1. In-vitro-Verwendung eines Polypeptids, das durch ein Polynukleotid codiert wird, das ausgewählt wird aus einem von (a) bis (d):
(a) ein Polynukleotid, welches ein Polypeptid codiert, das die Aminosäuresequenz von SEQ ID NO:2 umfasst;
(b) ein Polynukleotid, welches eine Codierungsregion der Nukleotidsequenz von SEQ ID NO:1 umfasst;
(c) ein Polynukleotid, welches mit einer DNA unter stringenten Bedingungen hybridisiert, die die Nukleotidsequenz von SEQ ID NO:1 umfasst;
(d) ein Polynukleotid, welches ein Fragment eines Polypeptids codiert, das eine Aminosäuresequenz von SEQ ID NO:2 umfasst;
als Histamin bindender Rezeptor.

## Revendications

1. Utilisation in vitro d'un polypeptide codé par un polynucléotide choisi parmi l'un quelconque de (a) à (d) :
(a) un polynucléotide qui code pour un polypeptide comprenant la séquence d'acides aminés de SEQ ID NO : 2 ;
(b) un polynucléotide qui comprend une région codante de la séquence nucléotidique de SEQ ID NO : 1 ;
(c) un polynucléotide qui s'hybride à un ADN comprenant la séquence nucléotidique de SEQ ID NO : 1 dans une condition stringente ;
(d) un polynucléotide qui code pour un fragment d'un polypeptide comprenant la séquence d'acides aminés de SEQ ID NO : 2 ;
en tant que récepteur de liaison de l'histamine.
